# EUROPEAN PATENT APPLICATION

(11) **EP 4 772 538 A1**
(43) Date of publication of application: **08.07.2026**
(21) Application number: 24857720.7
(22) Date of filing: 20.03.2024
(51) Int. Cl.: C07K 16/46, A61K 39/395, A61P 35/00

(54) **ANTIBODIES BINDING TO HLA-A02/MAGE-A4 COMPLEX AND USES THEREOF**

(30) Priority: 01.09.2023 CN 202311125718
(71) Applicant: Beijing Wisdomab Biotechnology Co., Ltd, Beijing 101111 (CN)
(72) Inventor: ZHANG, Xueping, Beijing 100176 (CN); LIU, Zhigang, Beijing 100039 (CN); ZHOU, Xiaowei, Beijing 100039 (CN); LIU, Yulan, Beijing 100176 (CN); HAO, Xiaobo, Beijing 100163 (CN); HU, Junjie, Beijing 100176 (CN); ZHANG, Suhua, Beijing 102600 (CN)
(74) Representative: Müller, Christian Stefan Gerd
(86) International application number: PCT/CN2024/082696
(87) International publication number: WO 2025/044168

(57) **Abstract**

The present application provides a bispecific antibody comprising a first antigen-binding fragment that binds to an HLA-A02/MAGE-A4 complex and/or an HLA-A02/MAGE-A8 complex and a second antigen-binding fragment that binds to an activated T cell antigen, a single-domain antibody that binds to an HLA-A02/MAGE-A4 complex and/or an HLA-A02/MAGE-A8 complex, a pharmaceutical composition comprising the bispecific antibody or the single-domain antibody, and uses thereof.

## Description

### CROSS REFERECE OF RELATED APPLICATION

The present application claims priority to Chinese Patent Application No. 202311125718.9, filed on September 1, 2023, which is incorporated herein by reference in its entirety.

### TECHNICAL FIELD

The present invention pretains to the field of genetic engineering and biomedicine, and more particularly, relates to a bispecific antibody that binds to an HLA-A02/MAGE-A4 complex or an HLA-A02/MAGE-A8 complex, a single-domain antibody that binds to the HLA-A02/MAGE-A4 complex or the HLA-A02/MAGE-A8 complex, and uses of the bispecific antibody or the single-domain antibody.

### BACKGROUND

Melanoma-associated antigen A (MAGE-A), belonging to the type I MAGE family, is an important member of the cancer-testis-associated antigens ^{[1]}. The MAGE-A gene family is located in the q28 region of the X chromosome and comprises 12 members (MAGE-A1 to MAGE-A12) ^{[2]}. All MAGE-A members possess the same MAGE homology domain (MHD), a highly conserved domain consisting of approximately 170 amino acids with high sequence homology to one another [3]

The melanoma associated antigen 4 protein (MAGE-A4) is a member of the MAGE-A gene family. MAGE-A4 is located in the cytoplasm. Studies have found that MAGE-A4 is also detected in the nucleus.

The function of MAGE-A4 is unclear. MAGE-A4 may be involved in cell cycle progression, transcriptional control, cell survival, or apoptosis. In malignant tumor, overexpressed MAGE-A4 may be associated with tumor growth and metastasis, and poor prognosis in patients.

The expression pattern of MAGE-A4 protein is consistent with that of the MAGE-A family proteins, being restrictively expressed in local testicular tissue, partially expressed in ovarian and placental tissues, and abnormally overexpressed in cancers ^{[4,5]}. Clinical histological identification shows that positive MAGE-A4 has been detected in many cancers, including urothelial carcinoma, carcinoma of bladder, lung cancer, ovarian cancer, esophageal squamous cell carcinoma, oral squamous cell carcinoma, or the like ^{[6-11]}.

As intracellular proteins, MAGE-A4 can be degraded in the proteasome, processed and presented on the cell surface via the major histocompatibility complex I (MHC I), where as a T-cell antigen, it is recognized by a T-cell receptor (TCR) to induce an immune response. For example, the polypeptide MAGE-A4₂₃₀₋₂₃₉ (GVYDGREHTV) derived from MAGE-A4 is presented on the cell surface in a complexe form with HLA-A02, triggering T-cell recognition and specifically killing tumor cells in a histocompatibility complex (MHC)-dependent manner ^{[12]}. Given the limitations of MAGE-A4 expression, it may be a potential molecular target for tumor diagnosis and immunotherapy [13, 14]

Bispecific antibodies (BsAbs), which simultaneously bind to both T-cell surface CD3 and target cell (e.g., tumor cell) surface antigen, establish an interaction between the target cells and the T-cells, causing cytotoxic T-cell activation and lysating the target cells in a non-histocompatibility complex (MHC)-dependent manner. These bispecific antibodies show promising potential in the treatment of various cancers. T-cell receptor-like antibodies (TCR-like antibodies), also known as TCR mimic (TCRm) antibodies, can effectively mimic TCRs and recognize MHC complexes of cell-surface tumor-specific antigen peptides. TCRm antibodies that bind to intracellular proteins of the MAGE-A4 class may serve as part of the CD3 bispecific antibodies that bind to the target antigen. Such bispecific antibodies may be more attractive due to the restriction of the target antigen expression.

In 2020, there were 19.29 million new cancer cases worldwide, among which 4.57 million new cancer cases were in China, accounting for 23.7% of the total. The global cancer death cases in 2020 reached 9.96 million, including 1.8 million (18%) from lung cancer, 0.92 million (9.2%) from colorectal carcinoma, 0.83 million (8.3%) from liver cancer, 0.77 million (7.7%) from gastric cancer, 0.68 million (6.8%) from breast cancer, 0.54 million (5.4%) from esophageal cancer, 0.47 million (4.7%) from pancreatic cancer, 0.38 million (3.8%) from prostate cancer, 0.34 million (3.4%) from cervical cancer, and 0.31 million (3.1%) from leukemia, accounting for 70.4% of the total ^{[15]}. Most of these cancers do not have good therapy. Therefore, there is an urgent need for new therapeutic strategies and drugs. MAGE-A4, an intracellular antigen expressed in a variety of solid tumors, may become a new target for solid tumor therapy.

Based on clinical needs, the exploration and development of bispecific antibodies targeting MAGE-A4-MHC have important clinical significance.

### SUMMARY OF THE INVENTION

In a first aspect, the present application provides a bispecific antibody comprising a first antigen-binding fragment that binds to an HLA-A02/MAGE-A4 complex and/or an HLA-A02/MAGE-A8 complex and a second antigen-binding fragment that binds to an activated T-cell antigen.

In some embodiments of the first aspect, the bispecific antibody is capable of mediating T cell activation by targeting HLA-A02⁺/MAGE-A4⁺ tumor cells, and/or the bispecific antibody is capable of mediating HLA-A02⁺/MAGE-A4⁺ tumor cell killing by PBMCs.

In some embodiments of the first aspect, the first antigen-binding fragment comprises HCDR1 as set forth in SEQ ID NO: 32, HCDR2 as set forth in SEQ ID NO: 33, and HCDR3 as set forth in SEQ ID NO: 34; wherein the amino acid sequences of the HCDRs are defined according to Kabat numbering scheme.

In some embodiments of the first aspect, the first antigen-binding fragment is in the form of a single-domain antibody.

In some embodiments of the first aspect, the first antigen-binding fragment comprises a monovalent or multivalent single-domain antibody that binds to the HLA-A02/MAGE-A4 complex and/or the HLA-A02/MAGE-A8 complex.

In some embodiments of the first aspect, the second antigen-binding fragment comprises HCDR1 as set forth in SEQ ID NO: 37, HCDR2 as set forth in SEQ ID NO: 38, HCDR3 as set forth in SEQ ID NO: 39, LCDR1 as set forth in SEQ ID NO: 40, LCDR2 as set forth in SEQ ID NO: 41, and LCDR3 as set forth in SEQ ID NO: 42; wherein the amino acid sequences of the HCDRs and LCDRs are defined according to Kabat numbering scheme.

In some embodiments of the first aspect, the second antigen-binding fragment is in the form of a single-chain variable fragment (scFv) or a Fab fragment.

In some embodiments of the first aspect, the first antigen-binding fragment comprises a divalent single-domain antibody that binds to the HLA-A02/MAGE-A4 complex and/or the HLA-A02/MAGE-A8 complex; optionally, the first antigen-binding fragment comprises two heavy chain variable regions of monovalent single-domain antibodies that bind to the HLA-A02/MAGE-A4 complex and/or the HLA-A02/MAGE-A8 complex.

In some embodiments of the first aspect, the first antigen-binding fragment comprises the amino acid sequence as set forth in SEQ ID NOs: 35, 36, 71, or 72; and/or the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments, the first antigen-binding fragment comprises the amino acid sequence as set forth in SEQ ID NO: 35; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments, the first antigen-binding fragment comprises the amino acid sequence as set forth in SEQ ID NO: 36; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments, the first antigen-binding fragment comprises the amino acid sequence as set forth in SEQ ID NO: 71; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments, the first antigen-binding fragment comprises the amino acid sequence as set forth in SEQ ID NO: 72; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment and the second antigen-binding fragment are linked via an Fc fragment of an antibody heavy chain constant region comprising a first Fc fragment and a second Fc fragment, wherein the first Fc fragment comprises the amino acid C at position 354 and the amino acid W at position 366; or the amino acid C at position 349, the amino acid S at position 366, the amino acid A at position 368, and the amino acid V at position 407; and the second Fc fragment comprises the amino acid C at position 354 and the amino acid W at position 366; or the amino acid C at position 349, the amino acid S at position 366, the amino acid A at position 368, and the amino acid V at position 407; wherein the amino acid positions of the antibody constant regions are determined according to EU numbering scheme. In some embodiments of the first aspect, the first antigen-binding fragment and the second antigen-binding fragment are linked via an Fc fragment of an antibody heavy chain constant region comprising a first Fc fragment and a second Fc fragment, wherein the first Fc fragment and the second Fc fragment comprise the amino acid F at position 234, the amino acid E at position 235 and the amino acid S at position 331, respectively; wherein the amino acid positions of the antibody constant regions are determined according to EU numbering scheme.

In some embodiments of the first aspect, the first antigen-binding fragment and the second antigen-binding fragment are linked via an antibody heavy chain constant region Fc fragment comprising a first Fc fragment and a second Fc fragment, wherein one of the first Fc fragment and the second Fc fragment is linked to the first antigen-binding fragment and the other of the first Fc fragment and the second Fc fragment is linked to the second antigen-binding fragment.

In some embodiments of the first aspect, the bispecific antibody comprises a first arm that binds to the HLA-A02/MAGE-A4 complex and/or the HLA-A02/MAGE-A8 complex and a second arm that binds to an activated T-cell antigen, wherein the first arm comprises the amino acid sequence as set forth in SEQ ID NOs: 45 or 74; and the second arm comprises the amino acid sequence as set forth in SEQ ID NO: 46.

In a second aspect, the present application provides a single-domain antibody that binds to the HLA-A02/MAGE-A4 complex and/or the HLA-A02/MAGE-A8 complex, comprising HCDR1 as set forth in SEQ ID NO: 32, HCDR2 as set forth in SEQ ID NO: 33, and HCDR3 as set forth in SEQ ID NO: 34; wherein the amino acid sequences of the HCDRs are defined according to Kabat numbering scheme.

In some embodiments of the second aspect, the single-domain antibody comprises the amino acid sequence as set forth in SEQ ID NOs: 35, 36, 71, or 72.

In a third aspect, the present application provides a nucleic acid molecule encoding the bispecific antibody of the first aspect or the single-domain antibody of the second aspect.

In a fourth aspect, the present application provides a pharmaceutical composition comprising the bispecific antibody of the first aspect or the single-domain antibody of the second aspect, and a pharmaceutically acceptable excipient, diluent, or carrier.

In a fifth aspect, the present application provides use of the bispecific antibody of the first aspect, the single-domain antibody of the second aspect, or the pharmaceutical composition of the fourth aspect in the manufacture of a medicament for the prevention or treatment of an HLA-A02/MAGE-A4-positive tumor and/or an HLA-A02/MAGE-A8-positive tumor.

### BRIEF DESCRIPTION OF THE DRAWINGS

FIG. 1 shows the ELISA results demonstrating that the anti-HLA-A02/MAGE-A4 complex TCRm antibody N18D10-Fc binds to the recombinant protein MHC-P1.
FIG. 2 shows the results demonstrating that the anti-HLA-A02/MAGE-A4 complex TCRm antibody N18D10-Fc binds to the antigen peptide segment MAGE-A4₂₃₀₋₂₃₉-pulsed T2 cells.
FIG. 3 shows the results demonstrating that the anti-HLA-A02/MAGE-A4 complex TCRm antibody N18D10-Fc binds to the alanine-scanning mutant peptides of MAGE-A4₂₃₀₋₂₃₉ (GVYDGREHTV)-plused T2 cells.
FIG. 4 shows the ELISA results demonstrating that the TCRm antibody N18D10-Fc binds to the recombinant protein MHC-P1
FIG. 5 shows the ELISA results demonstrating that TCRm×CD3 bispecific antibodies simultaneously bind to both MHC-P1 and CD3.
FIG. 6 shows the results demonstrating that TCRm×CD3 bispecific antibodies mediate the activation of Jurkat-Dual cells via antigen peptide segment-pulsed T2 cells.
FIG. 7 shows the results demonstrating that TCRm×CD3 bispecific antibodies mediate the activation of Jurkat-Dual cells via HLA-A02⁺/ MAGE-A4⁺ tumor cells.
FIG. 8 shows the results demonstrating that TCRm×CD3 bispecific antibodies mediate the killing of antigen peptide-pulsed T2 cells via PBMCs.
FIG. 9 shows the results demonstrating that TCRm×CD3 bispecific antibodies mediate the killing of A375 cells via PBMCs.
FIG. 10 shows the results demonstrating that TCRm×CD3 bispecific antibody mediates the killing of multiple antigen-positive tumor cells via PBMCs.
FIG. 11 shows the results demonstrating that TCRm×CD3 bispecific antibody mediates the activation of Jurkat-Dual cells via alanine-scanning mutant peptide segments of MAGE-A4₂₃₀₋₂₃₉ (GVYDGREHTV)-pulsed T2 cells.
FIG. 12 shows the results demonstrating that TCRm×CD3 bispecific antibody mediates the activation of Jurkat-Dual cells via similar peptide segments-pulsed T2 cells.
FIG. 13 shows the results demonstrating that TCRm×CD3 bispecific antibody does not mediate the killing of antigen-negative tumor cells via PBMCs.
FIG. 14 shows the results demonstrating that TCRm×CD3 bispecific antibody inhibits the tumor cells A375 growth in hPBMC-reconstituted mice.

### DESCRIPTION OF SEQUENCE

SEQ ID NO: 1 shows the amino acid sequence of the antigen peptide MAGE-A4₂₃₀₋₂₃₉ derived from human *(Homo sapiens).*
SEQ ID NO: 2 shows the amino acid sequence of MAGE-A4-S1 (peptide 1 similar to MAGE-A4₂₃₀₋₂₃₉) derived from human *(Homo sapiens).*
SEQ ID NO: 3 shows the amino acid sequence of MAGE-A4-S2 derived from human *(Homo sapiens).*
SEQ ID NO: 4 shows the amino acid sequence of MAGE-A4-S3 derived from human *(Homo sapiens).*
SEQ ID NO: 5 shows the amino acid sequence of MAGE-A4-S4 derived from human *(Homo sapiens).*
SEQ ID NO: 6 shows the amino acid sequence of MAGE-A4-S5 derived from human *(Homo sapiens).*
SEQ ID NO: 7 shows the amino acid sequence of MAGE-A4-S6 derived from human *(Homo sapiens).*
SEQ ID NO: 8 shows the amino acid sequence of MAGE-A4-S7 derived from human *(Homo sapiens).*
SEQ ID NO: 9 shows the amino acid sequence of MAGE-A4-S8 derived from human *(Homo sapiens).*
SEQ ID NO: 10 shows the amino acid sequence of MAGE-A4-S9 derived from human *(Homo sapiens).*
SEQ ID NO: 11 shows the amino acid sequence of MAGE-A4-S10 derived from human *(Homo sapiens).*
SEQ ID NO: 12 shows the amino acid sequence of MAGE-A4-S11 derived from human *(Homo sapiens).*
SEQ ID NO: 13 shows the amino acid sequence of MAGE-A4-S12 derived from human *(Homo sapiens).*
SEQ ID NO: 14 shows the amino acid sequence of MAGE-A4-S13 derived from human *(Homo sapiens).*
SEQ ID NO: 15 shows the amino acid sequence of MAGE-A4-S14 derived from human *(Homo sapiens).*
SEQ ID NO: 16 shows the amino acid sequence of MAGE-A4-S15 derived from human *(Homo sapiens).*
SEQ ID NO: 17 shows the amino acid sequence of MAGE-A4-S16 derived from human *(Homo sapiens).*
SEQ ID NO: 18 shows the amino acid sequence of MAGE-A4-S17 derived from human *(Homo sapiens).*
SEQ ID NO: 19 shows the amino acid sequence of MAGE-A4-S18 derived from human *(Homo sapiens).*
SEQ ID NO: 20 shows the amino acid sequence of MAGE-A4-S19 derived from human *(Homo sapiens).*
SEQ ID NO: 21 shows the amino acid sequence of MAGE-A4-S20 derived from human *(Homo sapiens).*
SEQ ID NO: 22 shows the amino acid sequence of MAGE-A4-S21 derived from human *(Homo sapiens).*
SEQ ID NO: 23 shows the amino acid sequence of MAGE-A4-S22 derived from human *(Homo sapiens).*
SEQ ID NO: 24 shows the amino acid sequence of MAGE-A4-S23 derived from human *(Homo sapiens).*
SEQ ID NO: 25 shows the amino acid sequence of MAGE-A4-S24 derived from human *(Homo sapiens).*
SEQ ID NO: 26 shows the amino acid sequence of MAGE-A4-S25 derived from human *(Homo sapiens).*
SEQ ID NO: 27 shows the amino acid sequence of MAGE-A4-S26 derived from human *(Homo sapiens).*
SEQ ID NO: 28 shows the amino acid sequence of MAGE-A4-S27 derived from human *(Homo sapiens).*
SEQ ID NO: 29 shows the amino acid sequence of MAGE-A4-S28 derived from human *(Homo sapiens).*
SEQ ID NO: 30 shows the amino acid sequence of MAGE-A4-S29 derived from human *(Homo sapiens).*
SEQ ID NO: 31 shows the amino acid sequence of MAGE-A4-S30 derived from human *(Homo sapiens).*
SEQ ID NOs: 32-34 show the amino acid sequences of HCDR1, HCDR2, and HCDR3 of the heavy chain variable regions of camel *(Camelus)* single-domain antibodies N18D10 and N18D10-h16, respectively.
SEQ ID NO: 35 shows the amino acid sequence of the heavy chain variable region of camel (*Camelus*) single-domain antibody N18D10.
SEQ ID NO: 36 shows the amino acid sequence of the heavy chain variable region of the humanized single-domain antibody N18D10-h16.
SEQ ID NOs: 37-39 show the amino acid sequences of HCDR1, HCDR2, and HCDR3 of the heavy chain variable region of the anti-human CD3 antibody IMCR, respectively.
SEQ ID NOs: 40-42 show the amino acid sequences of LCDR1, LCDR2, and LCDR3 of the light chain variable region of the anti-human CD3 antibody IMCR, respectively.
SEQ ID NO: 43 shows the amino acid sequence of the heavy chain variable region of the anti-human CD3 antibody IMCR.
SEQ ID NO: 44 shows the amino acid sequence of the light chain variable region of the anti-human CD3 antibody IMCR.
SEQ ID NO: 45 shows the amino acid sequence of the arm 2N18D10-h16-G1m3-FcHIn1 in the bispecific antibody 2N18D10-h16+IMCR.
SEQ ID NO: 46 shows the amino acid sequence of the arm IMCR-anti-CD3-scFv-G1m3-FcKn1 in the bispecific antibodies 2N18D10+IMCR and 2N18D10-h16+IMCR.
SEQ ID NO: 47 shows the amino acid sequence of HLA-A02 complex (MHC-P1) of GVYDGREHTV.
SEQ ID NO: 48 shows the amino acid sequence of the human *(Homo sapiens)* MAGE-A4.
SEQ ID NO: 49 shows the amino acid sequence of the linker.
SEQ ID NO: 50 shows the nucleotide sequence of the primer PCal-CH2R.
SEQ ID NO: 51 shows the amino acid sequence of the human *(Homo sapiens)* CD3E extracellular domain (hCD3E).
SEQ ID NO: 52 shows the amino acid sequence of the human *(Homo sapiens)* CD3D extracellular domain (hCD3D).
SEQ ID NO: 53 shows the amino acid sequence of the His tag.
SEQ ID NO: 54 shows the amino acid sequence of the human *(Homo sapiens)* IgG1 antibody Fc segment (hFc).
SEQ ID NO: 55 shows the amino acid sequence of the mouse (*Mus musculus*) IgG2a antibody Fc segment (mFc).
SEQ ID NO: 56 shows the amino acid sequence of the human *(Homo sapiens)* IgG1 subtype antibody heavy chain constant region.
SEQ ID NO: 57 shows the amino acid sequence of the human IgG1 subtype antibody heavy chain constant region mutant IgG1H.
SEQ ID NO: 58 shows the amino acid sequence of the human IgG1 subtype antibody heavy chain constant region mutant IgG1K.
SEQ ID NO: 59 shows the amino acid sequence of the human IgG1 subtype antibody heavy chain constant region mutant IgG1m3-H.
SEQ ID NO: 60 shows the amino acid sequence of the human IgG1 subtype antibody heavy chain constant region mutant IgG1m3-K.
SEQ ID NO: 61 shows the amino acid sequence of the human IgG1 subtype antibody heavy chain constant region mutant IgG1m3-H1n1.
SEQ ID NO: 62 shows the amino acid sequence of the human IgG1 subtype antibody heavy chain constant region mutant IgG1m3-Kn1.
SEQ ID NO: 63 shows the amino acid sequence of the human *(Homo sapiens)* λ iostype light chain constant region.
SEQ ID NO: 64 shows the amino acid sequence of human *(Homo sapiens)* κ iostype light chain constant region.
SEQ ID NO: 65 shows the amino acid sequence of the heavy chain variable region (VH) of the germline gene antibody DP47.
SEQ ID NO: 66 shows the amino acid sequence of the light chain variable region (Vκ) of the germline gene antibody DP47.
SEQ ID NO: 67 shows the amino acid sequence of the α chain variable region (Vα) of the control TCR binding to HLA-A02/MAGE-4.
SEQ ID NO: 68 shows the amino acid sequence of the β chain variable region (Vβ) of the control TCR binding to HLA-A02/MAGE-4.
SEQ ID NO: 69 shows the amino acid sequence of the first binding arm TCRA-Des-G1m3-FcKn1 in the control bifunctional molecule IMC-C103C.
SEQ ID NO: 70 shows the amino acid sequence of TCRB-Des-G1m3-FcH1n1 in the control TCR.
SEQ ID NO: 71 shows the amino acid sequence of the tandemly linked heavy chain variable regions from two camel single-domain antibodies N18D10.
SEQ ID NO: 72 shows the amino acid sequence of the tandemly linked heavy chain variable regions from two camel single-domain antibodies N18D10-h16.
SEQ ID NO: 73 shows the amino acid sequence of the anti-human *(Homo sapiens)* CD3 single-chain antibody IMCR.
SEQ ID NO: 74 shows the amino acid sequence of 2N18D10-G1m3-FcHIn1 in the bispecific antibody 2N18D10+IMCR.
SEQ ID NO: 75 shows the amino acid sequence of the second binding arm IMCR-anti-CD3-scFv-TCRB-Des-G1m3-FcH1n1 in the control bifunctional molecule IMC-C103C.
SEQ ID NO: 76 shows the amino acid sequence of the heavy chain variable region (057G03VH) of the control antibody 057D03 binding to the HLA-A02/MAGE-A4 complex.
SEQ ID NO: 77 shows the amino acid sequence of the light chain variable region (057G03Vκ) of the control antibody 057D03 binding to the HLA-A02/MAGE-A4 complex.
SEQ ID NO: 78 shows the amino acid sequence of the first component 057G03-Fd-EE-IMCR-anti-CD3VK-G1m3-Kn1 in the anti-HLA-A02/MAGE-A4 complex and CD3 control bispecific antibody 057G03.
SEQ ID NO: 79 shows the amino acid sequence of the second component 057G03-Fd-EE-G1m3-FcH1n1 in the anti-HLA-A02/MAGE-A4 complex and CD3 control bispecific antibody 057G03.
SEQ ID NO: 80 shows the amino acid sequence of the third component 057G03VK+CK-RK in the anti-HLA-A02/MAGE-A4 complex and CD3 control bispecific antibody 057G03.
SEQ ID NO: 81 shows the amino acid sequence of the fourth component IMCR-anti-CD3VH-CK in the anti-HLA-A02/MAGE-A4 complex and CD3 control bispecific antibody 057G03.
SEQ ID NO: 82 shows the amino acid sequence of the alanine-scanning mutant peptide segment at position G1 of MAGE-A4₂₃₀₋₂₃₉.
SEQ ID NO: 83 shows the amino acid sequence of the alanine-scanning mutant peptide segment at position V2 of MAGE-A4₂₃₀₋₂₃₉.
SEQ ID NO: 84 shows the amino acid sequence of the alanine-scanning mutant peptide segment at position Y3 of MAGE-A4₂₃₀₋₂₃₉.
SEQ ID NO: 85 shows the amino acid sequence of the alanine-scanning mutant peptide segment at position D4 of MAGE-A4₂₃₀₋₂₃₉.
SEQ ID NO: 86 shows the amino acid sequence of the alanine-scanning mutant peptide segment at position G5 of MAGE-A4₂₃₀₋₂₃₉.
SEQ ID NO: 87 shows the amino acid sequence of the alanine-scanning mutant peptide segment at position R6 of MAGE-A4₂₃₀₋₂₃₉.
SEQ ID NO: 88 shows the amino acid sequence of the alanine-scanning mutant peptide segment at position E7 of MAGE-A4₂₃₀₋₂₃₉.
SEQ ID NO: 89 shows the amino acid sequence of the alanine-scanning mutant peptide segment at position H8 of MAGE-A4₂₃₀₋₂₃₉.
SEQ ID NO: 90 shows the amino acid sequence of the alanine-scanning mutant peptide segment at position T9 of MAGE-A4₂₃₀₋₂₃₉.
SEQ ID NO: 91 shows the amino acid sequence of the alanine-scanning mutant peptide segment at position V10 of MAGE-A4₂₃₀₋₂₃₉.
SEQ ID NO: 92 shows the amino acid sequence of the human IgG1 subtype antibody fragment mutant IgG1m3-FcH1n1.
SEQ ID NO: 93 shows the amino acid sequence of the human IgG1 subtype antibody fragment mutant IgG1m3-FcKn1.
SEQ ID NO: 94 shows the amino acid sequence of human *(Homo sapiens)* MAGE-A8.

### DETAILED DESCRIPTION OF THE INVENTION

The inventors of the present application prepared a bispecific antibody (e.g., TCRm bispecific antibodies) by genetically engineering a first antigen-binding fragment that binds to an HLA-A02/MAGE-A4 complex and/or an HLA-A02/MAGE-A8 complex, and a second antigen-binding fragment that binds to an activated T-cell antigen (e.g., CD3 molecule). The first antigen-binding fragment binds to the complex of MAGE-A4₂₃₀₋₂₃₉ (GVYDGREHTV) and HLA-A02 and/or the complex of MAGE-A8₂₃₂₋₂₄₁ (GLYDGREHSV) and HLA-A02 presented on the surface of a target cell (e.g., tumor cells), and the second antigen-binding fragment binds to the activated T-cell antigen (e.g., CD3 molecules), thereby establishing an interaction between the target cell and the T cell. This interaction causes cytotoxic T-cell activation and lysis of the target cell (e.g., tumor cells) in a histocompatibility complex (MHC)-dependent manner, achieving the purpose of treating a disease (e.g., tumors). In various aspects, the present application provides a novel bispecific antibody comprising a first antigen-binding fragment that binds to an HLA-A02/MAGE-A4 complex and/or an HLA-A02/MAGE-A8 complex and a second antigen-binding fragment that binds to an activated T-cell antigen (e.g., CD3 molecules), a single-domain antibody that binds to an HLA-A02/MAGE-A4 complex and/or an HLA-A02/MAGE-A8 complex, a nucleic acid molecule encoding the bispecific antibody or the single-domain antibody, a vector comprising the nucleic acid molecule, a host cell comprising the nucleic acid molecule or the vector, a method for preparing and purifying the bispecific antibody or the single-domain antibody, and medical and biological applications of the bispecific antibody or the single-domain antibody. According to the sequences of the bispecific antibody or single-domain antibody provided in the present application, these antibodies can be constructed as medicaments for clinical use in the prevention or treatment of a tumor.

Unless otherwise indicated, the inventions of the present application can be implemented using conventional molecular biology, microbiology, cell biology, biochemistry, and immunological techniques in the art.

Unless otherwise indicated, the terms used in the present application have the meanings commonly understood by those skilled in the art.

### Definition

The term "HLA-A02/MAGE-A4 complex" as used herein refers to a complex of HLA-A02 molecules and MAGE-A4. In a specific embodiment of the present application, "HLA-A02/MAGE-A4 complex" refers to an HLA-A02/MAGE-A4₂₃₀₋₂₃₉ complex of an HLA-A02 molecule and the amino acids at positions 230-239 (MAGE-A4₂₃₀₋₂₃₉) of MAGE-A4 as set forth in SEQ ID NO: 48.

The term "HLA-A02/MAGE-A8 complex" as used herein refers to a complex of HLA-A02 molecules and MAGE-A8. In a specific embodiment of the present application, "HLA-A02/MAGE-A8 complex" refers to HLA-A02/MAGE-A8₂₃₂₋₂₄₁ complex of HLA-A02 molecule and the amino acids at position 232-241 (MAGE-A8₂₃₂₋₂₄₁) of MAGE-A8 as set forth in SEQ ID NO: 94.

The term "activated T-cell antigen" as used herein refers to an antigenic determinant expressed on the surface of T lymphocytes, particularly cytotoxic T lymphocytes, which is capable of inducing T cell activation upon interaction with an antigen-binding molecule. In particular, the interaction between an antigen binding molecule and an activated T-cell antigen can induce T cell activation by triggering a signal transduction cascade through T-cell receptor complexes. In a particular aspect, the activated T-cell antigen is CD3.

The term "antibody" as used herein refers to an immunoglobulin molecule capable of specifically binding to a target via at least one antigen recognition site located in its variable region. Targets include, but are not limited to, carbohydrates, polynucleotides, lipids, polypeptides, and the like. An "antibody" as used herein includes not only an intact (*i.e.,* full-length) antibody, but also a binding fragment thereof (e.g., Fab, Fab', F(ab')₂, or Fv), a variant thereof, a fusion protein comprising antibody portions, a humanized antibody, a chimeric antibody, a bispecific antibody, a linear antibody, a single-chain variable fragment, a single-domain antibody, a multi-specific antibody (e.g., a bispecific antibody), and any other modified configurations of an immunoglobulin molecule comprising a desired specific antigen recognition site, including a glycosylated antibody variant, an amino acid sequence variant of an antibody, and a covalently modified antibody.

Typically, an intact or full-length antibody comprises two heavy chains and two light chains. Each heavy chain contains the heavy chain variable region (VH) and the first, second and third constant regions (CH1, CH2, and CH3). Each light chain contains the light chain variable region (VL) and the constant region (CL). A full-length antibody may belong to any immunoglobulin class, such as an IgD, IgE, IgG, IgA, or IgM (or any of the above subclasses), but it is not restricted to any specific type. Immunoglobulins are categorized based on the amino acid sequences of their heavy chain constant regions. Generally, there are five primary classes, *i.e.,* IgA, IgD, IgE, IgG, and IgM, some of which are further divided into subclasses (subtypes), such as IgG1, IgG2, IgG3, IgG4, IgA1, and IgA2. The heavy chain constant regions corresponding to these individual immunoglobulin classes are referred to as α, δ, ε, γ, and µ, respectively. Subunit structures and three-dimensional conformations of different types of immunoglobulins are well characterized.

The term "bispecific antibody" as used herein refers to an antibody that has dual binding capacities for two antigen epitopes. The two antigen epitopes can be on different antigens or on the same antigen. Bispecific antibodies can have a variety of structural configurations. For example, a bispecific antibody may consist of two Fc fragments and two binding moieties fused to them, respectively (similar to a native antibody, except that the two arms bind to different antigen targets or epitopes), and the antigen-binding moieties can be single-domain antibodies, single-chain fragment antibodies (scFvs), or Fab fragments. When two non-overlapping epitopes of the same antigen are targeted, two different binding portions of the bispecific antibody each bind to the N-terminus of one Fc fragment, and the antigen-binding portions of the two arms can be in one of four configurations: single-domain antibody+Fab fragment, single-domain antibody+scFv, scFv+single-domain antibody, and Fab fragment+single-domain antibody. The Fc fragment may contain one or more mutations that ensures heavy chain heteropolymerization, and the KIH technique (knob-in-hole, KIH) is a strategy to address this heavy chain heteropolymerization. Generally, the KIH technique means that by modifying the amino acid sequence of the CH3 region to form a structure that facilitates pairing of heterodimeric hemiantibodies, the structure of a normal antibody can be maintained as much as possible while forming the bispecific antibody. For guidance on the KIH technique, see, for example, "An efficient route to human bispecific IgG", A. Margaret Merchant et al., Nature Biotechnology, Volume 16, 1998, which is incorporated herein by reference in its entirety.

The term "binding portion" or "binding fragment" as used herein is used interchangeably to refer to the portion or region of an intact antibody molecule responsible for binding to an antigen. The antigen-binding domain may comprise a heavy chain variable region (VH), a light chain variable region (VL), or both. Each of the VH and VL regions typically contains three complementarity determining regions (CDR1, CDR2, and CDR3).

It is well known to those skilled in the art that complementarity determining regions (CDRs, usually including CDR1, CDR2 and CDR3) are the regions of a variable region that have the greatest impact on the affinity and specificity of an antibody. The CDR sequences of VH or VL have two common schemes, namely, the Kabat numbering scheme and the Chothia numbering scheme (see, e.g., Kabat, "Sequences of Proteins of Immunological Interest", National Institutes of Health, Bethesda, Md. (1991); A1-Lazikani et al., J. Mol. Biol. 273:927-948 (1997); and Martin et al., Proc. Natl. Acad. Sci. USA 86:9268-9272 (1989)). For the sequences of the variable regions of a given antibody, the CDR sequences in VH and VL can be determined according to the Kabat or Chothia numbering scheme. In an embodiment of the present application, the CDR sequences are defined according to the Kabat numbering scheme.

For the sequences of the variable regions of a given antibody, the CDR sequences can be determined in a variety of ways, for example, using the online software Abysis (http://www.abysis.org/).

For typical antibodies, examples of an antigen-binding fragment include, but are not limited to, (1) an Fab fragment, which is a monovalent fragment consisting of a VL-CL chain and a VH-CH1 chain; (2) an F(ab')₂ fragment, which is a divalent fragment consisting of two Fab' fragments linked via a disulfide bridge in the hinge region (*i.e.,* a dimer of Fab'); (3) an Fv fragment consisting of VL and VH domains from a single antibody arm; (4) a single-chain Fv (scFv), which is a single polypeptide chain consisting of a VH domain and a VL domain via a polypeptide linker; (5) (scFv)₂, which comprises two VH domains linked via a peptide linker and two VL domains that associate with the two VH domains via disulfide bridges; and (6) single-domain antibody forms.

In the construction of a bispecific antibody, "binding moieties" include, but are not limited to, single-domain antibody forms, Fab fragment forms, and/or single-chain variable fragment (scFv) forms.

The term "single-chain variable fragment (scFv)" as used herein refers to a single-chain antibody generally constructed using genetic engineering techniques, comprising a polypeptide chain that contains a heavy chain variable region (VH) and a light chain variable region (VL). A flexible linker is typically incorporated between the heavy chain variable region and the light chain variable region to enable the heavy chain variable region and the light chain variable region to fold into a correct antigen-binding conformation.

The term "Fab (fragment antigen binding) fragment", "Fab portion" or a similar term as used herein refers to an antibody fragment capable of binding to an antigen that is produced after papain digestion of an intact antibody comprising a complete light chain (VL-CL), a heavy chain variable region, and a CH1 fragment (VH-CH1).

The term "single-domain antibody" as used herein refers to the variable domain of a naturally occurring heavy chain antibody that lacks light chains, comprising a heavy chain variable region (VHH) and conventional CH2 and CH3 regions (e.g., one or two groups (heavy chain variable region (VHH) and conventional CH2 and CH3 regions)). The separately cloned and expressed VHH structure maintains structural stability comparable to its parent heavy chain antibody and retains antigen-binding activity, representing the smallest known antigen-binding unit. Single-domain antibodies are also known as Nanobodies (Nb).

The terms "F_{c} fragment", "Fc domain", and "Fc portion" as used herein are used interchangeably to refer to the portion of an antibody heavy chain constant region, which includes the hinge region, CH2 domain, and CH3 domain as defined according to the EU numbering scheme for human IgG1 antibodies.

The term "specific binding" as used herein refers to the selective binding reaction between two molecules, such as the binding of an antibody to an epitope.

The term "tumor" as used herein refers to a neoplasm or solid lesion formed by abnormal cell growth. The tumor can be benign, premalignant, or malignant.

The term "malignant tumor" as used herein refers to or describes a physiological condition of a mammal, typically characterized by unregulated cell growth. Exemplary malignant tumors include cancer, solid tumors, melanoma, sarcoma, hematological malignancies, germinoma, and blastoma. More specific examples of malignant tumor include esophageal cancer, urothelial carcinoma, oral carcinoma, sarcoma, head and neck cancer, bladder carcer, melanoma, ovarian cancer, colorectal carcinoma, breast cancer, renal cell carcinoma, lung cancer including small cell lung cancer, non-small cell lung cancer, pulmonary adenocarcinoma, and pulmonary squamous cell carcinoma, cervical cancer, hepatic carcinoma, gastric cancer including gastrointestinal cancer, prostate cancer, pancreatic cancer, peritoneal cancer, hepatocellular carcinoma, glioblastoma, liver cancer, urinary tract cancer, hepatoma, endometrial/uterine cancer, salivary gland cancer, squamous cell carcinoma (e.g., squamous epithelial cell carcinoma), vulvar cancer, thyroid cancer, anal cancer, penile cancer, multiple myeloma, B-cell lymphoma, brain cancer, and metastases of the above cancers.

The term "hematological tumor" as used herein refers to a tumor arising from uncontrolled growth and proliferation of abnormal cells, typically originating in the bone marrow where blood cells are produced. Exemples of hematological tumors include various types of leukemia, multiple myeloma, and malignant lymphoma. More specific examples of hematological tumors include acute lymphoblastic leukemia (ALL), chronic lymphocytic leukemia (CLL), acute myeloid leukemia (AML), chronic myeloid leukemia (CML), hairy cell leukemia (HCL), T-cell prolymphocytic leukemia, large granule lymphocytic leukemia, juvenile myelomonocytic leukemia, B-cell prolymphocytic leukemia, Burkitt leukemia, adult T-cell leukemia, non-Hodgkin lymphoma, B-cell lymphoma, small lymphocytic lymphoma, lymphoplasmacytic lymphoma, primary macroglobulinemia (Waldenström macroglobulinemia), splenic marginal zone lymphoma, plasmacytoma, extranodal marginal zone B-cell lymphoma, MALT lymphoma, nodal marginal zone B-cell lymphoma (NMZL), follicular lymphoma, mantle cell lymphoma, diffuse large B-cell lymphoma, mediastinal (thymus) large B-cell lymphoma, intravascular large B-cell lymphoma, primary effusion lymphoma, Burkitt lymphoma, B-cell chronic lymphocytic lymphoma, classical Hodgkin lymphoma, nodular lymphocyte-predominant Hodgkin lymphoma, adult T-cell lymphoma, extranodal NK/T-cell lymphoma (nasal type), Enteropathy-associated T-cell lymphoma, hepatosplenic T-cell lymphoma, NK-cell lymphoblastic lymphoma, mycosis fungoides, Sézary syndrome, primary cutaneous CD30+ T-cell lymphoproliferative disease, primary cutaneous anaplastic large-cell lymphoma, lymphomatoid papulosis, angioimmunoblastic T-cell lymphoma, peripheral T-cell lymphoma not otherwise specified, and anaplastic large-cell lymphoma.

The term "solid tumor" as used herein refers to a tangible mass that can be detected by clinical examinations such as radiography (X-ray), computed tomography (CT), ultrasonography, or palpation. Solid tumors that have been diagnosed and treated clinically are classified into two categories, malignant and benign. Malignant solid tumors include: esophageal cancer (e.g. esophageal squamous cell carcinoma), urothelial carcinoma, oral carcinoma (e.g. oral squamous cell carcinoma), sarcoma (e.g. synovial sarcoma), head and neck cancer, liver cancer, lung cancer, bladder cancer, melanoma including metastatic melanoma, ovarian cancer, colorectal carcinoma, breast cancer, pediatric Hodgkin lymphoma subtypes including lymphocyte-predominant, nodular sclerosis, mixed cell subtype, lymphocyte-depleted; pediatric non-Hodgkin lymphoma: prolymphoblastic lymphoma, small non-cleaved cell lymphoma (Burkitt/non-Burkitt lymphoma), diffuse large B-cell lymphoma, anaplastic large cell lymphoma, and the like; pediatric renal tumors: nephroblastoma (Wilms tumor), renal clear cell carcinoma, rhabdoid tumor, renal clear cell sarcoma, renal primitive neuroectodermal tumor, and the like; pediatric neuroblastoma: neuroblastoma, ganglioneuroblastoma, ganglioneuroma; pediatric extracranial germinoma: mature teratoma, immature teratoma, endodermal sinus tumor (yolk sac tumor), seminoma, dysgerminoma, choriocarcinoma, embryonic carcinoma, and the like; osteosarcoma and chondrosarcoma; pediatric rhabdomyosarcoma: embryonal, alveolar, pleomorphic, and the like; soft tissue sarcomas: fibrosarcoma, malignant fibrous histiocytoma, liposarcoma, leiomyosarcoma, angiosarcoma, lymphangiosarcoma, malignant schwannoma, acinar soft tissue sarcoma, epithelioid sarcoma, clear cell sarcoma, malignant melanoma, synovial sarcoma, desmoplastic small round cell tumor, and the like; Ewing sarcoma family tumors: Ewing sarcoma, primitive neuroectodermal tumor; pediatric liver tumors: hepatoblastoma (embryonal, fetal, undifferentiated subtypes), hepatocellular carcinoma; retinoblastoma; other tumors: medulloblastoma of the posterior cranial fossa, nasopharyngeal carcinoma, papillary thyroid carcinoma, thymoma, pulmonary blastoma, pancreatoblastoma, islet cell tumor, ileocecal carcinoid, mesothelioma, and the like. Benign solid tumors include lymphangiomas, hemangiomas, thyroglossal cysts, and the like.

In a first aspect, the present application provides a bispecific antibody comprising a first antigen-binding fragment that binds to an HLA-A02/MAGE-A4 complex and/or an HLA-A02/MAGE-A8 complex and a second antigen-binding fragment that binds to an activated T-cell antigen.

In some embodiments of the first aspect, the bispecific antibody is capable of mediating the activation of T cells via HLA-A02⁺/ MAGE-A4⁺ tumor cells, and/or the bispecific antibody is capable of mediating the killing of HLA-A02⁺/ MAGE-A4⁺ tumor cells via PBMCs.

In some embodiments of the first aspect, the bispecific antibody is capable of mediating the activation of Jurkat-Dual cells via HLA-A02⁺/ MAGE-A4⁺ tumor cells.

In some embodiments of the first aspect, the first antigen-binding fragment binds to HLA-A02/ MAGE-A4 complex at an epitope comprising one or more amino acids at positions 230-239 of MAGE-A4 as set forth in SEQ ID NO: 48. In some embodiments, the amino acid sequence at positions 230-239 of MAGE-A4 as set forth in SEQ ID NO: 48 is GVYDGREHTV (SEQ ID NO: 1).

In some embodiments of the first aspect, the first antigen-binding fragment binds to HLA-A02/ MAGE-A4 complex at an epitope comprising at least one of amino acids at positions 232, 234, 235, 237, and 239 of MAGE-A4 as set forth in SEQ ID NO: 48. In some specific embodiments, the first antigen-binding fragment binds to the HLA-A02/MAGE-A4 complex at an epitope comprising amino acids at positions 232, 234, 235, 237, and 239 of MAGE-A4 as set forth in SEQ ID NO: 48. In some embodiments, the amino acids at positions 232, 234, 235, 237, and 239 of MAGE-A4 as set forth in SEQ ID NO: 48 correspond to the amino acids at positions 3, 5, 6, 8, and 10 of the MAGE-A4₂₃₀₋₂₃₉ (GVYDGREHTV) polypeptide as set forth in SEQ ID NO: 1.

In some embodiments of the first aspect, the first antigen-binding fragment binds to HLA-A02/MAGE-A8 complex at an epitope comprising amino acids at positions 232-241 of MAGE-A8 as set forth in SEQ ID NO: 94. In some embodiments, the amino acid sequence at positions 232-241 of MAGE-A8 as set forth in SEQ ID NO: 94 is GLYDGREHSV (SEQ ID NO: 2).

In some embodiments of the first aspect, the activated T-cell antigen is a CD3 molecule.

In some embodiments of the first aspect, the first antigen-binding fragment comprises HCDR1 as set forth in SEQ ID NO: 32, HCDR2 as set forth in SEQ ID NO: 33, and HCDR3 as set forth in SEQ ID NO: 34; wherein the amino acid sequences of the HCDRs are defined according to Kabat numbering scheme.

In some embodiments of the first aspect, the first antigen-binding fragment is in the form of a single-domain antibody.

In some embodiments of the first aspect, the first antigen-binding fragment comprises a monovalent or multivalent (e.g., 1, 2, 3, 4, 5, or 6-valent) single-domain antibody that binds to the HLA-A02/MAGE-A4 complex.

In some embodiments of the first aspect, the first antigen-binding fragment comprises a divalent single-domain antibody that binds to HLA-A02/ MAGE-A4 complex. In some embodiments, the first antigen-binding fragment comprises two heavy chain variable regions of monovalent single-domain antibodies that bind to the HLA-A02/MAGE-A4 complex. In some embodiments, the first antigen-binding fragment comprises two heavy chain variable regions of monovalent single-domain antibodies that bind to the HLA-A02/MAGE-A4 complex, linked via a linker. In some embodiments, the linker comprises a GS flexible peptide, such as (GGGGS)ₙ, where n is an integer ≥ 1. In some specific embodiments, the linker is GGGGSGGGGSGGGGS (SEQ ID NO: 49).

In some embodiments of the first aspect, the first antigen-binding fragment comprises a monovalent or multivalent (e.g., 1, 2, 3, 4, 5, or 6-valent) single-domain antibody that binds to HLA-A02/MAGE-A8 complex.

In some embodiments of the first aspect, the first antigen-binding fragment comprises a divalent single-domain antibody that binds to HLA-A02/ MAGE-A8 complex. In some embodiments, the first antigen-binding fragment comprises two heavy chain variable regions of monovalent single-domain antibodies that bind to HLA-A02/MAGE-A8 complex. In some embodiments, the first antigen-binding fragment comprises two heavy chain variable regions of monovalent single-domain antibodies that bind to HLA-A02/MAGE-A8 complex, linked via a linker. In some embodiments, the linker comprises a GS flexible peptide, such as (GGGGS)ₙ, where n is an integer ≥ 1. In some specific embodiments, the linker is GGGGSGGGGSGGGGS (SEQ ID NO: 49).

In some embodiments of the first aspect, the first antigen-binding fragment comprises the amino acid sequence as set forth in SEQ ID NO: 35.

In some embodiments of the first aspect, the first antigen-binding fragment comprises the amino acid sequence as set forth in SEQ ID NO: 36.

In some embodiments of the first aspect, the first antigen-binding fragment comprises the amino acid sequence as set forth in SEQ ID NO: 71.

In some embodiments of the first aspect, the first antigen-binding fragment comprises the amino acid sequence as set forth in SEQ ID NO: 72.

In some embodiments of the first aspect, the amino acid sequence as set forth in SEQ ID NO: 71 comprises two amino acid sequences as set forth in SEQ ID NO: 35.

In some embodiments of the first aspect, the amino acid sequence as set forth in SEQ ID NO: 72 comprises two amino acid sequences as set forth in SEQ ID NO: 36.

In some embodiments of the first aspect, the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 35; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 36; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 71; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 72; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises an amino acid sequence differing from the amino acid sequence as set forth in SEQ ID NOs: 35, 36, 71, or 72 by about 1, 2, 3, 4, 5, 6, 7, 8, 9, or 10 amino acid substitutions, deletions, and/or additions.

In some embodiments of the first aspect, the first antigen-binding fragment comprises an amino acid sequence having at least 90%, 91%, 92%, 93%, 94%, 95%, 96%, 97%, 98%, 99%, or higher identity to the amino acid sequence as set forth in SEQ ID NOs: 35, 36, 71, or 72.

In some embodiments of the first aspect, the amino acid sequence as set forth in SEQ ID NOs: 35, 36, 71, or 72 can be truncated at the C-terminus or N-terminus by about 1, 2, 3, 4, 5, 6, 7, 8, 9, 10, 11, 12, 13, 14, 15, 20, 25, or more amino acids while still retaining similar function of the first antigen-binding fragment.

In some embodiments of the first aspect, the amino acid sequence as set forth in SEQ ID NOs: 35, 36, 71, or 72 can have 1, 2, 3, 4, 5, 6, 7, 8, 9, 10, 11, 12, 13, 14, 15, 20, 25, or more amino acids added at the C-terminus or N-terminus, and the resulting amino acid sequence still retains similar function of the first antigen-binding fragment.

In some embodiments of the first aspect, the amino acid sequence as set forth in SEQ ID NOs: 35, 36, 71, or 72 can have 1, 2, 3, 4, 5, 6, 7, 8, 9, 10, 11, 12, 13, 14, 15, 20, 25, or more amino acids added or deleted at regions other than the C-terminus or N-terminus, provided that the altered amino acid sequence substantially retains similar function similar of the first antigen-binding fragment.

In some embodiments of the first aspect, the heavy chain variable region of the second antigen-binding fragment comprises the amino acid sequence as set forth in SEQ ID NO: 43.

In some embodiments of the first aspect, the heavy chain variable region of the second antigen-binding fragment comprises an amino acid sequence differing from the amino acid sequence as set forth in SEQ ID NO: 43 by about 1, 2, 3, 4, 5, 6, 7, 8, 9, or 10 amino acid substitutions, deletions, and/or additions.

In some embodiments of the first aspect, the heavy chain variable region of the second antigen-binding fragment comprises an amino acid sequence having at least 90%, 91%, 92%, 93%, 94%, 95%, 96%, 97%, 98%, 99%, or higher identity to the amino acid sequence as set forth in SEQ ID NO: 43.

In some embodiments of the first aspect, the amino acid sequence as set forth in SEQ ID NO: 43 can be truncated at the C-terminus or N-terminus by about 1, 2, 3, 4, 5, 6, 7, 8, 9, 10, 11, 12, 13, 14, 15, 20, 25, or more amino acids while still retaining similar function of the heavy chain variable region of the second antigen-binding fragment.

In some embodiments of the first aspect, the amino acid sequence as set forth in SEQ ID NO: 43 can have 1, 2, 3, 4, 5, 6, 7, 8, 9, 10, 11, 12, 13, 14, 15, 20, 25, or more amino acids added at the C-terminus or N-terminus, and the resulting amino acid sequence still retains similar function of the heavy chain variable region of the second antigen-binding fragment.

In some embodiments of the first aspect, the amino acid sequence as set forth in SEQ ID NO: 43 can have 1, 2, 3, 4, 5, 6, 7, 8, 9, 10, 11, 12, 13, 14, 15, 20, 25, or more amino acids added or deleted at regions other than the C-terminus or the N-terminus, provided that the altered amino acid sequence substantially retains similar function of the heavy chain variable region of the second antigen-binding fragment.

In some embodiments of the first aspect, the light chain variable region of the second antigen-binding fragment comprises the amino acid sequence as set forth in SEQ ID NO: 44.

In some embodiments of the first aspect, the light chain variable region of the second antigen-binding fragment comprises an amino acid sequence differing from the amino acid sequence as set forth in SEQ ID NO: 44 by about 1, 2, 3, 4, 5, 6, 7, 8, 9, or 10 amino acid substitutions, deletions, and/or additions.

In some embodiments of the first aspect, the light chain variable region of the second antigen-binding fragment comprises an amino acid sequence having at least 90%, 91%, 92%, 93%, 94%, 95%, 96%, 97%, 98%, 99%, or higher identity to the amino acid sequence as set forth in SEQ ID NO: 44.

In some embodiments of the first aspect, the amino acid sequence set forth in SEQ ID NO: 44 can be truncated at the C-terminus or N-terminus by about 1, 2, 3, 4, 5, 6, 7, 8, 9, 10, 11, 12, 13, 14, 15, 20, 25, or more amino acids, while still retaining similar function of the light chain variable region of the second antigen-binding fragment.

In some embodiments of the first aspect, the amino acid sequence set forth in SEQ ID NO: 44 can have 1, 2, 3, 4, 5, 6, 7, 8, 9, 10, 11, 12, 13, 14, 15, 20, 25, or more amino acids added at the C-terminus or N-terminus, and the resulting amino acid sequence still retains similar function of the light chain variable region of the second antigen-binding fragment.

In some embodiments of the first aspect, the amino acid sequence set forth in SEQ ID NO: 44 can have 1, 2, 3, 4, 5, 6, 7, 8, 9, 10, 11, 12, 13, 14, 15, 20, 25, or more amino acids added or deleted at regions other than the C-terminus or the N-terminus, provided that the altered amino acid sequence substantially retains similar function of the light chain variable region of the second antigen-binding fragment.

In some embodiments of the first aspect, the first antigen-binding fragment and the second antigen-binding fragment are linked via an Fc fragment of a heavy chain constant region comprising a first Fc fragment and a second Fc fragment, wherein the first Fc fragment comprises the amino acid C at position 354 and the amino acid W at position 366; or the amino acid C at position 349, the amino acid S at position 366, the amino acid A at position 368, and the amino acid V at position 407; and the second Fc fragment comprises the amino acid C at position 354 and the amino acid W at position 366; or the amino acid C at position 349, the amino acid S at position 366, the amino acid A at position 368, and the amino acid V at position 407; wherein the amino acid positions of the antibody constant regions are determined according to EU numbering scheme.

In some embodiments of the first aspect, the first antigen-binding fragment and the second antigen-binding fragment are linked via an Fc frag,emt of a heavy chain constant comprising a first Fc fragment and a second Fc fragment, wherein the first Fc fragment comprises the amino acid C at position 354 and the amino acid W at position 366, and the second Fc fragment comprises the amino acid C at position 349, the amino acid S at position 366, the amino acid A at position 368, and the amino acid V at position 407; wherein the amino acid positions of the antibody constant regions are determined according to EU numbering scheme.

In some embodiments of the first aspect, the first antigen-binding fragment and the second antigen-binding fragment are linked via Fc fragment of a heavy chain constant region comprising a first Fc fragment and a second Fc fragment, wherein amino acids at positions 234, 235, and 331 of the first Fc fragment and the second Fc fragment are F, E, and S, respectively; wherein the first Fc fragment and the second Fc fragment comprise the amino acid F at position 234, the amino acid E at position 235 and the amino acid S at position 331, respectively.

In some embodiments of the first aspect, the first antigen-binding fragment and the second antigen-binding fragment are linked via an antibody heavy chain constant region Fc fragment comprising a first Fc fragment and a second Fc fragment, wherein one of the first Fc fragment and the second Fc fragment is linked to the first antigen-binding fragment and the other of the first Fc fragment and the second Fc fragment is linked to the second antigen-binding fragment.

In some embodiments of the first aspect, the first antigen-binding fragment (e.g., at the C-terminus) is linked to the N-terminus of the first Fc fragment (e.g., an amino acid sequence such as SEQ ID NO: 92).

In some embodiments of the first aspect, the second antigen-binding fragment (e.g., at the C-terminus) is linked to the N-terminus of the second Fc fragment (e.g., an amino acid sequence such as SEQ ID NO: 93).

In some embodiments of the first aspect, the antibody heavy chain constant region Fc fragment is an Fc fragment of IgG1 subtype. In some embodiments, the first Fc fragment is an Fc fragment of IgG1 subtype; and/or the second Fc fragment is an Fc fragment of IgG1 subtype.

In some embodiments of the first aspect, the antibody heavy chain constant region Fc fragment is an Fc fragment of IgG1m3 allotype. In some embodiments, the first Fc fragment is an Fc fragment of IgG1m3 allotype; and/or the second Fc fragment is an Fc fragment of IgG1m3 allotype.

In some embodiments of the first aspect, the bispecific antibody comprises a first arm that binds to the HLA-A02/MAGE-A4 complex and a second arm that binds to the activated T-cell antigen, wherein the first arm comprises the amino acid sequence as set forth in SEQ ID NOs: 45 or 74; and the second arm comprises the amino acid sequence as set forth in SEQ ID NO: 46.

In some embodiments of the first aspect, the bispecific antibody comprises a first arm that binds to the HLA-A02/MAGE-A8 complex and a second arm that binds to an activated T-cell antigen, wherein the first arm comprises the amino acid sequence as set forth in SEQ ID NOs: 45 or 74; and the second arm comprises the amino acid sequence as set forth in SEQ ID NO: 46.

In some embodiments of the first aspect, the first arm comprises the amino acid sequence as set forth in SEQ ID NOs: 45 or 74.

In some embodiments of the first aspect, the first arm comprises the amino acid sequence differing from the amino acid sequence as set forth in SEQ ID NOs: 45 or 74 by about 1, 2, 3, 4, 5, 6, 7, 8, 9, or 10 amino acid substitutions, deletions, and/or additions.

In some embodiments of the first aspect, the first arm comprises an amino acid sequence having at least 90%, 91%, 92%, 93%, 94%, 95%, 96%, 97%, 98%, 99%, or higher identity to the amino acid sequence set forth in SEQ ID NOs: 45 or 74.

In some embodiments of the first aspect, the amino acid sequence as set forth in SEQ ID NOs: 45 or 74 can be truncated at the C-terminus or N-terminus by about 1, 2, 3, 4, 5, 6, 7, 8, 9, 10, 11, 12, 13, 14, 15, 20, 25, or more amino acids, while still retaining similar function of the first arm.

In some embodiments of the first aspect, the amino acid sequence set forth in SEQ ID NOs: 45 or 74 can have 1, 2, 3, 4, 5, 6, 7, 8, 9, 10, 11, 12, 13, 14, 15, 20, 25, or more amino acids added at the C-terminus or N-terminus, and the resulting amino acid sequence still retains similar function of the first arm.

In some embodiments of the first aspect, the amino acid sequence as set forth in SEQ ID NOs: 45 or 74 can have 1, 2, 3, 4, 5, 6, 7, 8, 9, 10, 11, 12, 13, 14, 15, 20, 25, or more amino acids added or deleted at regions other than the C-terminus or N-terminus, provided that the altered amino acid sequence substantially retains similar function of the first arm.

In some embodiments of the first aspect, the second arm comprises the amino acid sequence as set forth in SEQ ID NO: 46.

In some embodiments of the first aspect, the second arm comprises an amino acid sequence differing from the amino acid sequence as set forth in SEQ ID NO: 46 by about 1, 2, 3, 4, 5, 6, 7, 8, 9, or 10 amino acid substitutions, deletions, and/or additions.

In some embodiments of the first aspect, the second arm comprises an amino acid sequence having at least 90%, 91%, 92%, 93%, 94%, 95%, 96%, 97%, 98%, 99%, or higher identity to the amino acid sequence as set forth in SEQ ID NO: 46.

In some embodiments of the first aspect, the amino acid sequence set forth in SEQ ID NO: 46 can be truncated at the C-terminus or N-terminus by about 1, 2, 3, 4, 5, 6, 7, 8, 9, 10, 11, 12, 13, 14, 15, 20, 25, or more amino acids, while still retaining similar function of the second arm.

In some embodiments of the first aspect, the amino acid sequence as set forth in SEQ ID NO: 47 can have 1, 2, 3, 4, 5, 6, 7, 8, 9, 10, 11, 12, 13, 14, 15, 20, 25, or more amino acids added at the C-terminus or N-terminus, and the resulting amino acid sequence still retains similar function of the second arm.

In some embodiments of the first aspect, the amino acid sequence set forth in SEQ ID NO: 47 can have 1, 2, 3, 4, 5, 6, 7, 8, 9, 10, 11, 12, 13, 14, 15, 20, 25, or more amino acids added or deleted at a region other than the C-terminus or the N-terminus, provided that the altered amino acid sequence substantially retains similar function of the second arm.

In a second aspect, the present application provides a single-domain antibody that binds to an HLA-A02/MAGE-A4 complex and/or an HLA-A02/MAGE-A8 complex, comprising HCDR1 as set forth in SEQ ID NO: 32, HCDR2 as set forth in SEQ ID NO: 33, and HCDR3 as set forth in SEQ ID NO: 34; wherein the amino acid sequences of the HCDRs are defined according to Kabat numbering scheme.

In some embodiments of the second aspect, the single-domain antibody comprises the amino acid sequence as set forth in SEQ ID NO: 35.

In some embodiments of the second aspect, the single-domain antibody comprises the amino acid sequence as set forth in SEQ ID NO: 36.

In some embodiments of the second aspect, the single-domain antibody comprises the amino acid sequence as set forth in SEQ ID NO: 71.

In some embodiments of the second aspect, the single-domain antibody comprises the amino acid sequence as set forth in SEQ ID NO: 72.

In some embodiments of the second aspect, the single-domain antibody comprises an amino acid sequence differing from the amino acid sequence as set forth in SEQ ID NOs: 35, 36, 71, or 72 by about 1, 2, 3, 4, 5, 6, 7, 8, 9, or 10 amino acid substitutions, deletions, and/or additions.

In some embodiments of the second aspect, the single-domain antibody comprises an amino acid sequence having at least 90%, 91%, 92%, 93%, 94%, 95%, 96%, 97%, 98%, 99%, or higher identity to SEQ ID NOs: 35, 36, 71, or 72.

In some embodiments of the second aspect, the amino acid sequence set forth in SEQ ID NOs: 35, 36, 71, or 72 can be truncated at the C-terminus or N-terminus by about 1, 2, 3, 4, 5, 6, 7, 8, 9, 10, 11, 12, 13, 14, 15, 20, 25, or more amino acids, while still retaining similar function of the single-domain antibody.

In some embodiments of the second aspect, the amino acid sequence set forth in SEQ ID NOs: 35, 36, 71 or 72 can have 1, 2, 3, 4, 5, 6, 7, 8, 9, 10, 11, 12, 13, 14, 15, 20, 25, or more amino acids added at the C-terminus or N-terminus, and the resulting amino acid sequence still retains similar function of the single-domain antibody.

In some embodiments of the second aspect, the amino acid sequence set forth in SEQ ID NOs: 35, 36, 71, or 72 can have 1, 2, 3, 4, 5, 6, 7, 8, 9, 10, 11, 12, 13, 14, 15, 20, 25, or more amino acids added or deleted at regions other than the C-terminus or the N-terminus, provided that the altered amino acid sequence substantially retains similar function of the single-domain antibody.

In a third aspect, the present application provides a nucleic acid molecule encoding the bispecific antibody of the first aspect or the single-domain antibody of the second aspect.

In some embodiments of the third aspect, the nucleic acid molecule may include a DNA molecule and an RNA molecule. The nucleic acid molecule can be single-stranded or double-stranded and can be cDNA.

In some embodiments of the third aspect, the nucleic acid molecule is operably linked to a regulatory amino acid sequence that can be recognized by a host cell transformed with a vector.

In a fourth aspect, the present application provides a pharmaceutical composition comprising the bispecific antibody of the first aspect or the single-domain antibody of the second aspect, and a pharmaceutically acceptable excipient, diluent, or carrier.

In some embodiments of the fourth aspect, the pharmaceutical composition is used for the prevention or treatment of an HLA-A02/MAGE-A4-positive tumor.

In some embodiments of the fourth aspect, the HLA-A02/MAGE-A4-positive tumor is selected from the group consisting of esophageal cancer (e.g., esophageal squamous cell carcinoma), urothelial carcinoma, oral carcinoma (e.g., oral squamous cell carcinoma), sarcoma (e.g., synovial sarcoma), head and neck cancer, lung cancer, liver cancer, bladder cancer, melanoma (e.g., metastatic melanoma), ovarian cancer, colorectal carcinoma, and breast cancer.

In some embodiments of the fourth aspect, the pharmaceutical composition is used for the prevention or treatment of an HLA-A02/MAGE-A8-positive tumor.

In some embodiments of the fourth aspect, the HLA-A02/MAGE-A8-positive tumor is selected from the group consisting of esophageal cancer (e.g., esophageal squamous cell carcinoma), urothelial carcinoma, oral carcinoma (e.g., oral squamous cell carcinoma), sarcoma (e.g., synovial sarcoma), head and neck cancer, lung cancer, liver cancer, bladder cancer, melanoma (e.g., metastatic melanoma), ovarian cancer, colorectal carcinoma, and breast cancer.

In some embodiments of the fourth aspect, the pharmaceutical composition may further comprise one or more of the following: a lubricant, such as talc, magnesium stearate, and mineral oil; a wetting agent; an emulsifier; a suspending agent; a preservative such as benzoic acid, sorbic acid and calcium propionate; a sweetener and/or a flavoring agent, and the like.

In some embodiments of the fourth aspect, the pharmaceutical composition of the present application can be formulated as a tablet, a pill, a powder, a lozenge, an elixir, a suspension, an emulsion, a solution, a syrup, a suppository, a capsule, and the like.

In some embodiments of the fourth aspect, the pharmaceutical composition of the present application can be delivered using any physiologically acceptable administration route which includes, but is not limited to, oral administration, parenteral administration, nasal administration, rectal administration, intraperitoneal administration, intravascular injection, subcutaneous administration, transdermal administration, inhalation administration, or the like.

In some embodiments of the fourth aspect, the pharmaceutical composition for therapeutic uses can be formulated for storage in the form of a lyophilized formulation or an aqueous solution by mixing a reagent of desired purity with a pharmaceutically acceptable carrier or excipient, as appropriate.

In a fifth aspect, the present application provides use of the bispecific antibody of the first aspect, the single-domain antibody of the second aspect, or the pharmaceutical composition of the fourth aspect in the manufacture of a medicament for the prevention or treatment of an HLA-A02/MAGE-A4-positive tumor and/or an HLA-A02/MAGE-A8-positive tumor.

In some embodiments of the fifth aspect, the HLA-A02/MAGE-A4-positive tumor is selected from the group consisting of esophageal cancer (e.g., esophageal squamous cell carcinoma), urothelial carcinoma, oral carcinoma (e.g., oral squamous cell carcinoma), sarcoma (e.g. synovial sarcoma), head and neck cancer, lung cancer, liver cancer, bladder cancer, melanoma (metastatic melanoma), ovarian cancer, colorectal carcinoma, and breast cancer.

In some embodiments of the fifth aspect, the HLA-A02/MAGE-A8-positive tumor is selected from the group consisting of esophageal cancer (e.g., esophageal squamous cell carcinoma), urothelial carcinoma, oral carcinoma (e.g., oral squamous cell carcinoma), sarcoma (e.g., synovial sarcoma), head and neck cancer, lung cancer, liver cancer, bladder cancer, melanoma (metastatic melanoma), ovarian cancer, colorectal carcinoma, and breast cancer

In a sixth aspect, the present application provides a method for preventing or treating an HLA-A02/MAGE-A4-positive tumor and/or an HLA-A02/MAGE-A8-positive tumor, comprising administering to a subject in need thereof the bispecific antibody of the first aspect, the single-domain antibody of the second aspect, or the pharmaceutical composition of the fourth aspect.

In some embodiments of the sixth aspect, the HLA-A02/MAGE-A4-positive tumor is selected from the group consisting of esophageal cancer (e.g., esophageal squamous cell carcinoma), urothelial carcinoma, oral carcinoma (e.g., oral squamous cell carcinoma), sarcoma (e.g. synovial sarcoma), head and neck cancer, lung cancer, liver cancer, bladder cancer, melanoma (metastatic melanoma), ovarian cancer, colorectal carcinoma, and breast cancer

In some embodiments of the sixth aspect, the HLA-A02/MAGE-A8-positive tumor is selected from the group consisting of esophageal cancer (e.g., esophageal squamous cell carcinoma), urothelial carcinoma, oral carcinoma (e.g., oral squamous cell carcinoma), sarcoma (e.g., synovial sarcoma), head and neck cancer, lung cancer, liver cancer, bladder cancer, melanoma (metastatic melanoma), ovarian cancer, colorectal carcinoma, and breast cancer

The present application further provides a vector comprising a nucleic acid molecule encoding the bispecific antibody of the first aspect, or the single-domain antibody of the second aspect, and a host cell comprising the nucleic acid molecule or vector. In other aspects, the application also provides a method for producing the bispecific antibody of the first aspect, or the single-domain antibody of the second aspect. In some embodiments, the method for producing the bispecific antibody of the first aspect or the single-domain antibody of the second aspect comprises culturing the host cell to facilitate expression of the nucleic acid molecule. In some embodiments, the method for producing the bispecific antibody of the first aspect, or the single-domain antibody of the second aspect, further comprises recovering the bispecific antibody or the single-domain antibody from the host cell culture medium.

It should be understood that the foregoing detailed description is intended only to enable those skilled in the art to better understand the present application and is not intended to limit it in any way. Various modifications and variations to the described embodiments can be made by those skilled in the art.

The following Examples are provided for purposes of illustration only and are not intended to limit the scope of the present application.

### Examples

### Example 1: Preparation of recombinant proteins

The following were prepared according to the reference ^{[16]}: the disulfide trap single-chain trimer (dtSCT) structure of MHC I complex of the antigen peptide MAGE-A4₂₃₀₋₂₃₉ (GVYDGREHTV), i.e., HLA-A02 complex of GVYDGREHTV (MHC-P1-His, SEQ ID NO: 47); as well as the human CD3E extracellular region (hCD3E, SEQ ID NO: 51) and the human CD3D extracellular region (hCD3D, SEQ ID NO: 52). These recombinant proteins all have many post-translational modifications (e.g., glycosylation or disulfide bonds), and thus a mammal cell expression system was used to maintain their structure and function. To facilitate protein purification and monoclonal antibody functional identification, a His tag (His, SEQ ID NO: 53), theFc fragment of human IgG1 (Fc, SEQ ID NO: 56) or the Fc fragment of murine IgG2a (mFc, SEQ ID NO: 55) was added to the C-terminus of the recombinant proteins. In the preparation of recombinant antibodies, the antibody heavy chain constant region can be the human IgG1 subtype (SEQ ID NO: 56) or its various variants, such as IgG1H (SEQ ID NO: 57), IgG1K (SEQ IDNO: 58), IgG1m3-H (SEQ ID NO: 59), IgG1m3-K (SEQ ID NO: 60), IgG1m3-H1n1 (SEQ ID NO: 61), or IgG1m3-Kn1 (SEQ ID NO: 62); and the antibody light chain constant region can be the human lambda (λ) isotype (SEQ ID NO: 63) or the human kappa (κ) isotype (SEQ ID NO: 64).

Genes including the His tag, mFc, or Fc coding gene for the various recombinant proteins described above were designed and synthesized according to the amino acid sequences of the recombinant proteins of interest in the Uniprot database. The synthesized genes for the various recombinant proteins were cloned into a suitable vector for eukaryotic expression (such as pcDNA3.1 from Invitrogen Inc.) using conventional techniques in molecular biology. Plasmids prepared for recombinant protein expression were then transfected with liposomes (such as 293fectin from Invitrogen Inc.) or other cationic transfection reagent (such as PEI, and the like) into HEK293 cells (such as HEK293F cells from invitrogen Inc.), which were cultured in suspension under a serum-free condition for 3-4 days. The culture supernatant was then harvested by centrifugation or the like.

Recombinant proteins expressed as fusions with His tags were subjected to one-step purification from the culture supernatant using a metal chelate affinity chromatography column (such as HisTrap FF from GE Inc.). Recombinant proteins expressed as fusions with mFc/Fc tags were subjected to one-step purification using a ProteinA/G affinity chromatography column (such as Mabselect SURE from GE Inc.). The preservation buffer for the recombinant proteins was then replaced with PBS (pH7.0) or another suitable buffer using a desalting column (such as Hitrap desaulting from GE Inc.). When necessary, the antibody samples can be sterilized by filtration, and then stored in aliquots at - 20°C.

### Example 2: Screening of antibodies binding to HLA-A02/MAGE-A4 complex from a camel immune library

### 2.1 Construction of camel immune library

One healthy adult bactrian camel was selected, and blood was collected to obtain baseline serum before immunization. For the first immunization, 1 mg of MHC-P1-His fusion protein was emulsified with Fischer's complete adjuvant and injected subcutaneously at multiple points. For booster immunizations every two weeks, 1 mg of MHC-P1-His fusion protein was emulsified with Fischer's incomplete adjuvant and injected subcutaneously at multiple points. A total of five booster immunizations were conducted, and blood samples were collected before each immunization for antibody titer analysis. For the seventh immunization, 1 mg of MHC-P1-His fusion protein without adjuvant was taken as an antigen and injected subcutaneously at multiple points for high-dose booster immunization. 200 mL of peripheral blood was collected for lymphocyte isolation 3 days after the last immunization.

Camel peripheral blood lymphocytes were isolated from 200 mL of peripheral blood using the Camel Peripheral Blood Lymphocyte Isolation Solution Kit (Solarbio, CAT#P5750). Total RNA was extracted from the isolated lymphocytes using Total RNA Extraction Kit (for Animal Cells) (TIANGEN Biotech (BEIJING) Co., Ltd., CAT#DP430). The extracted total RNA was used as a template to synthesize the camel single-domain antibody heavy chain variable region (VHH) using First-Strand cDNA Synthesis Kit (Thermo scientific, CAT#K1621). Gene-specific primers were used as primers for reverse transcription. The primers annealed to regions located in the CH2 domain of the antibody heavy chain constant region, with the sequence of PCal-CH2R as: TCCTTCCCCGTCAGCCAGTCCT (SEQ ID NO: 50). The synthesized cDNAs were immediately stored at -70°C for future use. Then, the cDNAs obtained by reverse transcription was used as a template to synthesize primers according to reference ^{[17]}, and the camel single-domain antibody VHH gene was amplified and isolated using nested PCR. Finally, the amplified VHH genes were cloned into the vector pADSCFV-S (see Chinese Patent Application No. 201510097117.0 ^{[18]}) to construct a VHH library. This antibody library had a capacity of 1.5E+08 with an accuracy of 75%.

### 2.2 Screening of camel immune library

The phage library displaying camel single-domain antibodies constructed in Section 2.1 was screened by a solid phase screening strategy (see, Phage Display: A Practical Approach, Clackson, T. (USA) and Lowman, H. B. (USA) (ed.), translated by Lan Ma et al., Chemical Industry Press, May 2008.5 ^{[19]} for the experimental protocol) using the recombinant MHC-P1-His prepared in Example 1 as antigens. Three rounds of screening were carried out by means of binding, elution, neutralization, infection and amplification. Finally, a TCR mimetic (TCRm) antibody N18D10 that specifically binds to MHC-P1-His was obtained.

By using conventional molecular biological methods, the nucleotide sequence encoding the N18D10 heavy chain variable region (SEQ ID NO: 35) was cloned into a eukaryotic expression vector (such as pcDNA3.1 from Invitrogen Inc.) containing the nucleotide sequence encoding the Fc region of the human IgG1 to express N18D10-Fc recombinant protein. Meanwhile, the monoclonal antibody DP47-IgG1 of DP47 (germline gene antibody, see U.S. Patent Application No. US 20160200833A1 ^{[20]}, with the amino acid sequence of DP7VH set forth in SEQ ID NO: 65 and the amino acid sequence of DP7Vκ set forth in SEQ ID NO: 66) was prepared as a negative control. According to patent application WO 2017175006A1 ^{[21]}, the Vα (TCRA, SEQ ID NO: 67) and Vβ (TCRB, SEQ ID NO: 68) variable region genes of IMC-C103C were synthesized and separately cloned into eukaryotic expression vectors containing the Fc fragment nucleotide sequence with Knob-mutation fused to Cα and the Fc fragment nucleotide sequence with Hole-mutation fused to Cβ. The resulting two chains, designated as TCRA-Des-G1m3-FcKn1 (SEQ ID NO: 69) and TCRB-Des-G1m3-FcH1n1 (SEQ ID NO: 70), were co-expressed to assemble the TCR control molecule that binds to MHC-P1, named P1-TCR.

### Example 3: Identification of TCRm antibodies binding to HLA-A02/MAGE-A4 complex

### 3.1 Affinity analysis of anti-HLA-A02/MAGE-A4 complex TCRm antibody

The affinity of the anti-HLA-A02/MAGE-A4 TCRm antibody N18D10-Fc was determined by the surface plasmon resonance technique (SPR) using Biacore T200. Related reagents and consumables, such as the Amine Coupling Kit (BR-1000-50), the Human Antibody Capture Kit (BR-1008-39), a Series S CM5 chip (14100530), and 10×HBS-EP (BR100669) at pH 7.4, were purchased from GE Healthcare. According to the kit instructions, the surface of the carboxylated CM5 chip was activated with 1-ethyl-3-(3-dimethylaminopropyl) carbodiimide hydrochlorid (EDC) and N-Hydroxysuccinimide (NHS). The capture antibodies, anti-human IgG (Fc), were diluted to 25 µg/mL with 10 mM sodium acetate, pH 5.0, and then injected at a flow rate of 10 µL/min to achieve a coupling amount of approximately 10,000 response units (RU). This was followed by injection of 1 M ethanolamine to block unreacted groups. For the kinetic measurement, the TCRm antibody N18D10-Fc was diluted to 1 µg/mL, and injected at 10 µL/min to ensure capture of approximately 60 RU by the anti-human Fc antibodies. MHC-P1-His was then serially diluted to concentrations of 1.23 nM, 3.74 nM, 11.1 nM, 33.3 nM, and 100 nM, and injected at 30 µL/min from lowest to highest concentration at 25°C, with a binding time of 90 seconds and a dissociation time of 1,200 seconds. 3 M MgCl₂ solution was injected at 10 µL/min for 30 seconds to regenerate the chip surface. The association rate (Kₐ) and dissociation rate (K_{d}) were calculated by fitting the binding and dissociation sensorgrams to a 1:1 binding model using Biacore T200 Evaluation Software (version 3.2.1). The dissociation equilibrium constant (K_{D}) was calculated as K_{d}/Ka. The results are shown in Table 1.

**Table 1. Affinity of anti-HLA-A02/MAGE-A4 complex TCRm antibody N18D10-Fc for MHC-P1**

| | Kₐ(M⁻¹s⁻¹) | K_{d}(s⁻¹) | K_{D}(M) |
|---|---|---|---|
| N18D10-Fc | 1.851E+5 | 7.176E-5 | 3.878E-10 |

### 3.2 Binding of anti-HLA-A02/MAGE-A4 complex TCRm antibody to recombinant protein MHC-P1

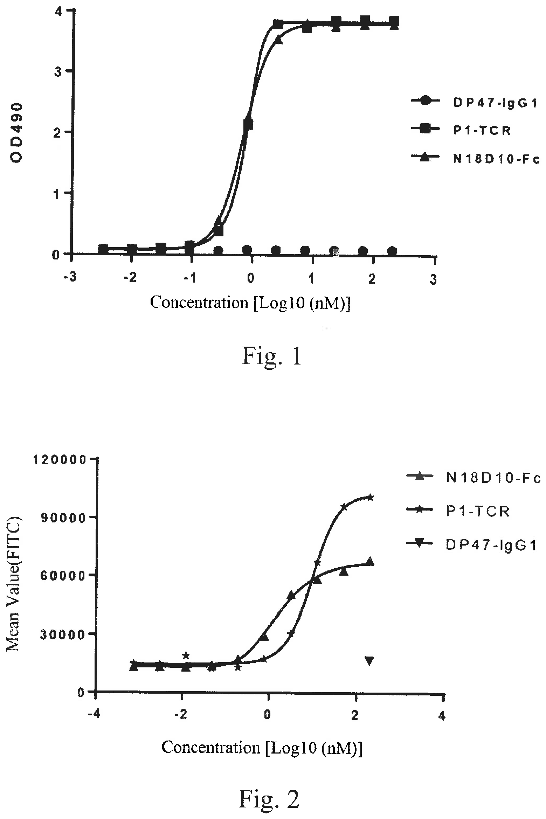

The anti-HLA-A02/MAGE-A4 complex TCRm antibody N18D10-Fc was coated on 96-well ELISA plates at 5 µg/mL, 100 µL per well, and incubated overnight at 4°C. After blocking with the blocking solution (PBS containing 0.1% Tween-20 and 3% skim milk) at 37°C for 1 hour, the recombinant protein MHC-P1-His was serially diluted with PBS at a starting concentration of 30 µg/mL (with 3-fold dilution gradients, for a total of 10 concentrations). 100 µL of each dilution was added into each well of the blocked 96-well ELISA plate and incubated at 37°C for 1 hour. The ELISA plates were washed with PBS containing 0.1% Tween 20, and then 100 µL of the HRP- Conjugated Anti-His-Tag Mouse Monoclonal Antibody (Beijing CoWin Biotech Co., Ltd, CW0285M) was added and incubated at 37°C for 1 hour. The ELISA plate was washed with PBS containig 0.1% Tween-20, and then OPD substrate developing solution was added. After 5-10 minutes, the color reaction was terminated with 1 M H₂SO₄, and optical density values were measured using a microplate reader at dual wavelengths of 492 nm/630 nm. FIG. 1 shows the results of N18D10-Fc specifically binds to MHC-P1.

### 3.3 Binding of anti-HLA-A02/MAGE-A4 complex TCRm antibody to antigen peptide-pulsed T2 cells

T2 cells (human lymphoma cells, HLA-A02⁺, Shanghai Honsun Biological Technology Co., Ltd) were harvested, centrifuged, and resuspended in growth medium to a concentration of 1 ×10⁶ cells/mL. Then, 1 mL of the suspension was plated into each well of a 24-well plate. The peptide segment MAGE-A4₂₃₀₋₂₃₉ (GVYDGREHTV) was chemically synthesized (GenScript Biotechnology Co., Ltd) and added to the 24-well plate at a final concentration of 50 µg/mL for overnight pulsing. After 16 hours, the cells were centrifuged and resuspended in PBS buffer containing 1% BSA to a concentration of 2×10⁶ cells/mL. 100 µL of the suspension was plated into each well of a 96-well V-bottom plate, and the supernatant was removed after centrifugation. The antibody N18D10-Fc, the positive control sample P1-TCR, and the negative control DP47-IgG 1 were prepared by diluting with PBS to a starting concentration of 200 nM, with 4-fold dilution gradients. 100 µL of each dilution was added to each well containing cells and incubated at 4°C for 1 hour. After washing the cells three times with 200 µL PBS, the cells were incubated with goat anti-human IgG-FITC (Beijing Zhong Shan-Golden Bridge Biological Technology CO., LTD, ZF-0308) at 100 µL per well for 30 minutes at 4°C in the dark. After washing the wells three times with 200 µL PBS, the cells were resuspended in 100 µL PBS and detected in the FITC channel by flow cytometry (ACEA, Novocyte). FIG. 2 shows the results of N18D10-Fc specifically binding to the antigen peptide MAGE-A4₂₃₀₋₂₃₉-pulsed T2 cells, indicating that N18D10-Fc binds to the HLA-A02/MAGE-A4 complex on the cell surface.

### 3.4 Binding of anti-HLA-A02/MAGE-A4 complex TCRm antibody to key amino acids of MAGE-A4

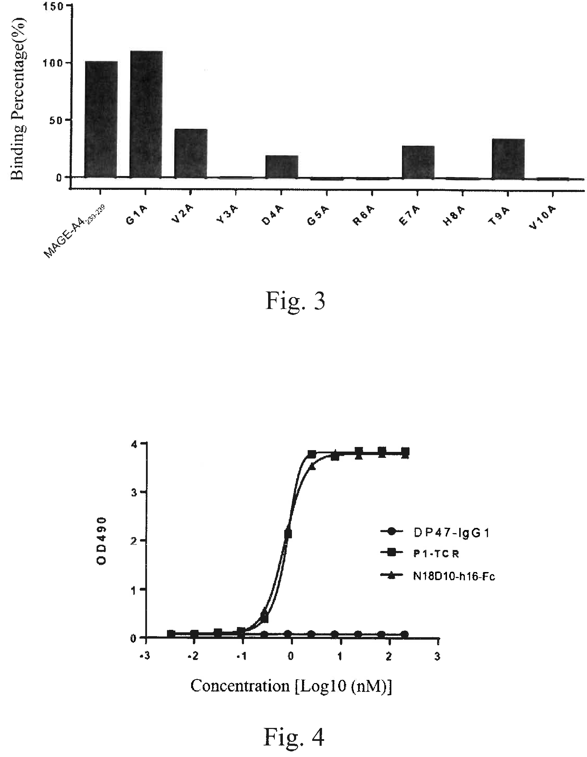

The alanine-scanning mutant peptide segments of MAGE-A4₂₃₀₋₂₃₉ (GVYDGREHTV) were chemically synthesized. Following the method described in Section 3.3, the binding of N18D10 to T2 cells pulsed with alanine-scanning mutant peptides of MAGE-A4₂₃₀₋₂₃₉ (GVYDGREHTV) was detected. T2 cells were harvested, centrifuged, and resuspended in growth medium to a concentration of 1×10⁶ cells/mL. Then, 1 mL of the suspension was plated into each well of a 24-well plate. The peptide segment MAGE-A4₂₃₀₋₂₃₉ (GVYDGREHTV) and its alanine-scanning mutant peptide segments were added to the 24-well plate at a final concentration of 50 µg/mL for overnight pulsing. After 16 hours, the cells were centrifuged and resuspended in PBS buffer containing 1% BSA to a concentration of 2×10⁶ cells/mL. 100 µL of the suspension was plated into each well of a 96-well V-bottom plate, and the supernatant was removed after centrifugation. Antibody N18D10-Fc was prepared by diluting with PBS to a starting concentration of 10 nM. 100 µL of each dilution was added to each well containing cells and incubated at 4°C for 1 hour. After washing the wells three times with 200 µL PBS, the cells were incubated with goat anti-human IgG-FITC at 100 µL per well for 30 minutes at 4°C in the dark. After washing the wells three times with 200 µL PBS, the cells were resuspended in 100 µL PBS and detected in the FITC channel by flow cytometry (ACEA, Novocyte). The binding of N18D10 to the alanine-scanning mutant peptide segments was calculated relative to that of the original peptide. Table 2 and FIG. 3 showed the results demonstrating that the binding capacity of the TCRm antibody N18D10 was decreased by more than 90% after the alanine substitution at positions 3, 5, 6, 8, or 10 of MAGE-A4₂₃₀₋₂₃₉(GVYDGREHTV), indicating that residues 3, 5, 6, 8, and 10 of MAGE-A4₂₃₀₋₂₃₉ (GVYDGREHTV) are key amino acids for N18D10 binding.

**Table 2. Binding of TCRm antibody N18D10 to alanine-scanning mutant peptide segments of MAGE-A4₂₃₀₋₂₃₉ (GVYDGREHTV)-pulsed T2 cells**

| | MAGE-A4₂₃₀₋₂₃₉ | G1A | V2A | Y3A | D4A | G5A | R6A | E7A | H8A | T9A | V10A |
|---|---|---|---|---|---|---|---|---|---|---|---|
| N18D10 | 100.00% | 109.51% | 41.06% | -0.12% | 18.40% | -1.02% | -0.56% | 27.44% | -0.13% | 33.62% | 0.35% |

### Example 4: Humanization and identification of anti-HLA-A02/MAGE-A4 complex TCRm antibody

### 4.1 Humanization of anti-HLA-A02/MAGE-A4 complex TCRm antibody

TCRm antibody N18D10 was humanized to reduce its immunogenicity. A classic framework grafting strategy ^{[22]} was used for the humanization scheme. The amino acid sequence of N18D10 was compared to the germline gene sequences of human antibodies in the IMGT database, respectively. Appropriate germline gene sequences were selected to provide the framework regions 1 to 3 of the antibodies (FR1+FR2+FR3), and the appropriate J region gene sequence was selected to provide the framework region 4 (FR4). This template can be selected based on a variety of factors, such as relative total length of the antibody, CDR size, amino acid residues at the junction between the antibody framework regions (FRs) and the hypervariable regions (CDRs), overall homology of sequences, and similar considerations. The selected template can be a mixture of multiple sequences or a consensus template, with the aim of maintaining the appropriate conformation of complementarity determining regions (CDRs) of the parent antibody as much as possible. At the same time, considering the solubility, stability, and expression yield of the humanized antibody, the four hot-spot amino acids 37F/44E/45R/47F in FR2 were reversely mutated, respectively, and the humanized molecule N18D10-h16 was finally obtained.

By using conventional molecular biological methods, the nucleotide sequence encoding the N18D10-h16 variable region (SEQ ID NO: 36) was cloned into a eukaryotic expression vector containing the nucleotide sequence encoding the Fc region of human IgG1(such as pcDNA3.1 from Invitrogen Inc.) to express N18D10-h16-Fc recombinant protein.

### 4.2 Affinity analysis of humanized anti-HLA-A02/MAGE-A4 complex TCRm antibody molecule

Following the method described in Section 3.1, the affinity of the recombinant anti-HLA-A02/MAGE-A4 complex TCRm antibody N18D10-h16-Fc was analyzed with Biacore T200. The results were shown in Table 3.

**Table 3. Affinity of anti-HLA-A02/MAGE-A4 complex TCRm antibody N18D10-h16-Fc for MHC-P1 complex**

| | Kₐ(M⁻¹s⁻¹) | K_{d}(s⁻¹) | K_{D}(M) |
|---|---|---|---|
| MHC-P1-His | 1.631E+5 | 7.610E-5 | 4.665E-10 |

### 4.3 Binding of anti-HLA-A02/MAGE-A4 complex TCRm antibody to MHC-P1

Following the method described in Section 3.2, the binding activity of the anti-HLA-A02/MAGE-A4 complex antibody N18D10-h16-Fc to the recombinant protein MHC-P1-His was determined. FIG. 4 shows the results demonstrating that N18D10-h16-Fc specifically binds to the recombinant protein MHC-P1-His, indicating that N18D10-h16-Fc binds to the HLA-A02/MAGE-A4 complex.

### Example 5: Preparation and identification of TCRm×CD3 bispecific antibody

### 5.1 Preparation of TCRm×CD3 bispecific antibody

The nucleotide sequences encoding the heavy chain variable regions of the divalent 2N18D10 or divalent 2N18D10-h16 single-domain antibody against the HLA-A02/MAGE-A4 complex and the nucleotide sequence encoding the single-chain antibody against CD3 (IMCR-anti-CD3-scFv, see anti-CD3 v9 in U.S. Patent Application No. US 582133A ^{[23]}) were separatelyy cloned into a suitable eukaryotic expression vector to co-express the bispecific antibody against both the HLA-A02/MAGE-A4 complex and CD3. Specifically, the nucleotide sequence encoding 2N18D10 (SEQ ID NO: 71) or 2N18D10-h16 (SEQ ID NO: 72) was cloned into a eukaryotic expression vector fused with the nucleotide sequence encoding the Fc fragment with the Hole mutation (IgG1m3-FcH1n1, SEQ ID NO: 92), and the nucleotide sequence encoding IMCR-anti-CD3-ScFv (SEQ ID NO: 73) was cloned into a eukaryotic expression vector fused with the nucleotide sequence encoding the Fc fragment with the Knob mutation (IgG1m3-FcKn1, SEQ ID NO: 93). Meanwhile, a DP47 bispecific antibody based on the same structure was prepared as a negative control.

The eukaryotic expression vector constructed for expressing 2N18D10-G1m3-FcHIn1 (SEQ ID NO: 74) or 2N18D10-h16-IgG1m3-FcH1n1 (SEQ ID NO: 45) and the eukaryotic expression vector for IMCR-anti-CD3-ScFv-G1m3-FcKn1 (SEQ ID NO: 46) were co-transfected into HEK293F cells using lipofectamine and cultured in serum-free medium for 3-5 days. The culture supernatant was collected by centrifugation and the bispecific antibody was purified using Protein A affinity chromatography (e.g., Mabselect SURE, GE Healthcare). Then the recombinant protein preservation buffer was exchanged to PBS (pH 7.0) or another suitable buffer using a desalting column (e.g., Hitrap Desaulting, GE Healthcare). The desalted protein solution was further purified by size-exclusion chromatography (SEC, Superdex200, GE Healthcare) to obtain the proteins of interest, named 2N18D10+IMCR and 2N18D10-h16+IMCR, respectively. If necessary, the antibody sample were sterilized by filtration and stored in aliquots at -20°C for further use.

According to Patent Application WO 2017175006A1, the single-chain antibody IMCR-anti-CD3-scFv was fused to the N-terminus of TCRB-Des-G1m3-FcH1n1, generating the TCR/CD3 bispecific molecule IMC-C103C, consisting of two chains, named TCRA-Des-G1m3-FcKn1 (SEQ ID NO: 69) and IMCR-anti-CD3-scFv-TCRB-Des-G1m3-FcH1n1 (SEQ ID NO: 75), respectively.

According to Patent Application WO 2021122875A1^{[24]}, the variable region sequences of 057D03 (the amino acid sequence of 057G03VH as set forth in SEQ ID NO: 76; the amino acid sequence of 057G03Vκ as set forth in SEQ ID NO: 77) were synthesized. The anti-HLA-A02/MAGE-A4 complex and anti-CD3 bispecific antibody 057G03 was prepared, consisting of four chains, named 057G03-Fd-EE-IMCR-anti-CD3VK-G1m3-Kn1 (SEQ ID NO: 78), 057G03-Fd-EE-G1m3-FcH1n1 (SEQ ID NO: 79), 057G03VK+CK-RK (SEQ ID NO: 80) and IMCR-anti-CD3VH-CK (SEQ ID NO: 81), respectively.

The expression and purification of IMC-C103C and 057G03 bispecific antibodies followed the same method as for 2N18D10+IMCR and 2N18D10-h16+IMCR. The final samples were sterilized by filtration and stored in aliquots at -20°C for further use.

### 5.2 Affinity analysis of TCRm×CD3 bispecific antibody

Following the method described in Section 3.1, the affinities of recombinant TCRm×CD3 bispecific antibodies 2N18D10+IMCR and 22N18D10-h16+IMCR were analyzed using Biacore T200. The results are shown in Tables 4 and 5.

**Table 4. Affinity of TCRm×CD3 bispecific antibody for MHC-P1**

| | Kₐ(M⁻¹s⁻¹) | K_{d}(s⁻¹) | K_{D}(M) |
|---|---|---|---|
| 2N18D10+IMCR | 1.961E+5 | 7.607E-5 | 3.880E-10 |
| 2N18D10h16+IMCR | 1.820E+5 | 7.834E-5 | 4.304E-10 |
| IMC-C103C | 8.520E+4 | 4.594E-5 | 5.392E-10 |
| 057G03 | 5.071E+5 | 1.672E-2 | 3.297E-8 |

**Table 5. Affinity of TCRm×CD3 bispecific antibody for CD3D-CD3E-mFc**

| | Kₐ(M⁻¹s⁻¹) | Kₐ(s⁻¹) | K_{D}(M) |
|---|---|---|---|
| 2N18D10+IMCR | 2.037E+5 | 2. 700E-4 | 1.325E-9 |
| 2N18D10h16+IMCR | 1.946E+5 | 2.681E-4 | 1.378E-9 |
| IMC-C103C | 2.582E+5 | 2.797E-4 | 1.083E-9 |
| 057G03 | 1.178E+5 | 2.699E-4 | 2.291E-9 |

### 5.3 TCRm×CD3 bispecific antibody simultaneously recognizes both antigens MHC-P1 and CD3

The TCRm×CD3 bispecific antibodies were detected to simultaneously bind to the bidirectional antigens CD3 and MHC-P1 by a conventional ELISA method.

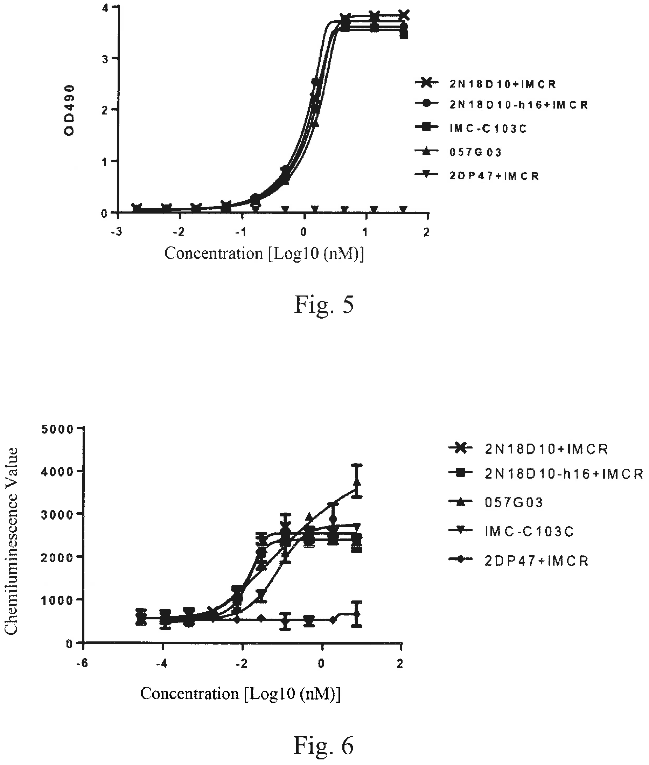

The antigen CD3D-CD3E-mFc was coated on 96-well ELISA plates (3 µg/mL, 100 µL per well) and incubated overnight at 4°C. Then the wells were blocked with blocking solution (PBS containing 0.1% Tween-20 and 3% skim milk) at 37°C for 1 hour. TCRm×CD3 bispecific antibodies were serially diluted with PBS at a starting concentration of 30 µg/mL (with 3-fold dilution gradients, for a total of 10 concentrations). 100 µL of each dilution was added into each well of the blocked 96-well ELISA plate and incubated at 37°C for 1 hour. The ELISA plates were washed with PBS containing 0.1% Tween-20, and then MHC-P1-His antigen (10 µg/mL) was added at 100 µL per well and incubated at 37°C for 1 hour. The ELISA plates were washed with PBS containing 0.1% Tween- 20, and then HRP-conjugated anti-His-tag mouse monoclonal antibody (Beijing CoWin Biotech Co., Ltd, CW0285M) was added and incubated at 37°C for 1 hour. The ELISA plate was washed with PBS containing 0.1% Tween 20, and OPD substrate developing solution was added. After 5-10 minutes, the color reaction was terminated with 1 M H₂SO₄, and optical density values were measured using a microplate reader at dual wavelengths of 492 nm/630 nm. The ELISA analysis results, as shown in FIG. 5, demonstrated that the TCRm×CD3 bispecific antibodies 2N18D10+IMCR and 2N18D10-h16+IMCR can simultaneously recognize both CD3 and MHC-P1 bidirectional antigens.

### 5.4 TCRm×CD3 bispecific antibody mediates activation of Jurkat-Dual cells

### 5.4.1 TCRm×CD3 bispecific antibody mediates activation of Jurkat-Dual cells via antigen peptide-pulsed T2 cells

T2 cells were harvested, centrifuged and resuspended in growth medium to a concentration of 1×10⁶ cells/mL. Then, 1 mL of the suspension was plated into each well of a 24-well plate. The peptide segment MAGE-A4₂₃₀₋₂₃₉ (GVYDGREHTV) was added to the 24-well plate at a final concentration of 50 µg/mL for overnight pulsing. After 16 hours, the cells were centrifuged and resuspended in RPMI 1640 medium to a concentration of 4×10⁵ cells/mL. Then, 50 µL of the suspension was plated into each well. Jurkat-Dual cells (Invivogen) were harvested, centrifuged and resuspended in RPMI 1640 medium to a concentration of 4×10⁵ cells/mL. Then, 50 µL of the suspension was added into each well of the T2 cells-seeded plate to obtain a final effector-to-target (E: T) ratio of 1:1. The bispecific antibodies 2N18D10+IMCR and 2N18D10-h16+IMCR, at a starting final concentration of 7.5 nM (with 4-fold dilution gradients, for a total of 8 concentrations), were then added at 100 µL per well. The negative control sample 2DP47+IMCR and the positive control samples 057G03 and IMC-C103C were used at the same concentrations. After 24 hours of incubation, the supernatant was collected, and the TCRm×CD3 bispecific antibodies were assayed for their ability to activate Jurkat-Dual cells via antigen peptide-pulsed T2 cells, according to the QUANTI-Luc^{™} instructions (QUANTI-Luc^{™}, Invivogen, rep-qlc2). The results showed that the bispecific antibodies, 2N18D10+IMCR and 2N18D10-h16+IMCR, could mediate the specificactivation of Jurkat-Dual cells via antigen peptide segment-pulsed T2 cells. Furthermore, compared with 057G03 and IMC-C103C, the bispecific antibodies 2N18D10+IMCR and 2N18D10h16+IMCR exhibited stronger activation effects on Jurkat-Dual cells (FIG. 6). The EC₅₀ values were shown in Table 6.

**Table 6. EC₅₀ values of TCRm×CD3 bispecific antibodies in mediating activation of Jurkat-Dual cells via exogenous antigen peptide segment-pulsed T2 cells**

| | 2N18D10+IMCR | 2N18D10-h16+IMCR | 057G03 | IMC-C103C |
|---|---|---|---|---|
| EC₅₀(n M) | 0.01446 | 0.01431 | 0.1746 | 0.0706 |

### 5.4.2 TCRm×CD3 bispecific antibody mediates the activation of Jurkat-Dual cells via HLA-A02⁺/MAGE-A4⁺ tumor cells

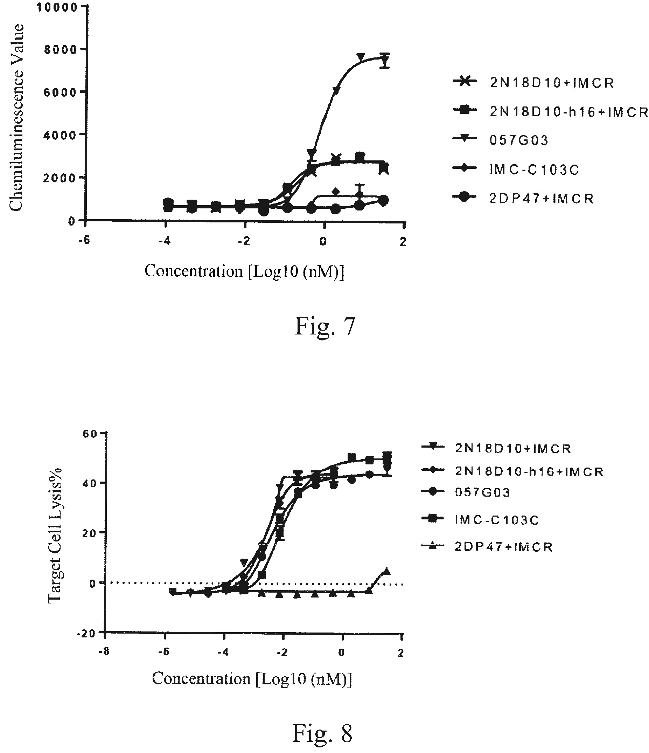

A375 cells (human malignant melanoma cells, HLA-A02⁺/MAGE-A4⁺, Nanjing Cobioer Biosciences Co., Ltd.) were harvested. After digestion and centrifugation, the cells were resuspended in RPMI 1640 medium to a concentration of 4×10⁵ cells/mL. Then, 50 µL of the suspension was plated into each well of a plate. Jurkat-Dual cells were harvested, centrifuged and resuspended in RPMI 1640 medium to a concentration of 4×10⁵ cells/mL. Then, 50 µL of the suspension was added into each well of the plate to obtain a final E:T ratio of 1:1. The bispecific antibodies 2N18D10+IMCR and 2N18D10-h16+IMCR, at a starting concentration of 30 nM (with 4-fold dilution gradients, for a total of 10 concentrations), were then added at 100 µL per well. The negative control sample 2DP47+IMCR and the positive control samples 057G03 and IMC-C103C were used at the same concentrations. After 48 hours of incubation, the supernatant was collected, and TCRm×CD3 bispecific antibodies were assayed for their ability to mediate the specific activation of Jurkat-Dual cells via tumor cells under different conditions, according to the QUANTI-Luc^{™}instructions (QUANTI-Luc^{™}, Invivogen, rep-qlc2). The results in FIG. 6 showed that the bispecific antibodies 2N18D10+IMCR and 2N18D10-h16+IMCR could mediate the specific activation of Jurkat-Dual cells via the HLA-A02⁺/MAGE-A4⁺ tumor cells A375. Furthermore, compared with 057G03 and IMC-C103C, the bispecific antibodies 2N18D10+IMCR and 2N18D10-h16+IMCR exhibited stronger activation effects on Jurkat-Dual cells (FIG. 7). The EC₅₀ values were shown in Table 7.

**Table 7. EC₅₀ values of TCRm×CD3 bispecific antibodies in mediating activation of Jurkat-Dual cells via HLA-A02⁺/MAGE-A4⁺ tumor cells**

| | 2N18D10+IMCR | 2N18D10h16+IMCR | 057G03 | IMC-C103C |
|---|---|---|---|---|
| EC₅₀(n M) | 0.1893 | 0.1446 | 0.7494 | 0.5203 |

### 5.5 TCRm×CD3 bispecific antibody mediates the specific killing of target cells via PBMCs

### 5.5.1 Isolation of human peripheral blood mononuclear cells (PBMCs)

Blood was collected from healthy volunteers (50 mL from each) who had signed informed consent. The inclusion criteria for volunteers were: age over 18 years; no HIV and HBV infection; normal results of blood routine test; and non-pregnant and non-lactating female.

PBMCs were isolated from the whole blood of the volunteers by Ficoll density gradient centrifugation and cultured in RPMI 1640 medium.

### 5.5.2 TCRm×CD3 bispecific antibody mediates the killing of antigen peptide-pulsed T2 cells via PBMCs

T2 cells were harvested, centrifuged and resuspended in growth medium to a concentration of 1×10⁶ cells/mL. Then, 1 mL of the suspension was plated into each well of a 24-well plate. The peptide segment MAGE-A4₂₃₀₋₂₃₉ (GVYDGREHTV) was added to the 24-well plate at a final concentration of 50 µg/mL for overnight pulsing. After 16 hours, the cells were centrifuged and resuspended in RPMI 1640 medium to a concentration of 4×10⁵ cells/mL. Then, 50 µL of the suspension was plated into each well of a 96-well cell plate. The bispecific antibodies 2N18D10+IMCR and 2N18D10-h16+IMCR, at a starting concentration of 30 nM (with 4-fold dilution gradients, for a total of 10 concentrations), were then added at 100 µL per well. The negative control sample 2DP47+IMCR and the positive control samples 057G03 and IMC-C103C were used at the same concentrations. PBMCs (effector cells) were resuspended to 4×10⁶ cells/mL. Then, 50 µL of the suspension was added into each well of the above cell plates, achieving a final E:T ratio of 10:1. A separate target cell control (antigen peptide-pulsed T2 cells), a separate effector cell control (PBMC), and a medium-only blank control were prepared. The volume of each well was adjusted to 200 µL with medium. After 24 hours of incubation, the supernatant was collected, and the TCRm×CD3 bispecific antibody-mediated killing of antigen peptide-pulsed T2 cells via PBMCs were analyzed according to the CytoTox96^{®} Non-Radioactive Cytotoxicity Assay instructions (promega, G1780). The results showed that the TCRm×CD3 bispecific antibodies 2N18D10+IMCR and 2N18D10-h16+IMCR could mediate the specific killing of antigen peptide-pulsed T2 cells via PBMCs. Compared with 057G03 and IMC-C103C, the bispecific antibodies 2N18D10+IMCR and 2N18D10h16+IMCR exhibited higher activity in mediating the killing of antigen peptide-pulsed T2 cells via PBMCs, as shown in Table 8 and FIG. 8.

**Table 8. EC₅₀ values of TCRm×CD3 bispecific antibodies in mediating killing of antigen peptide-pulsed T2 cells via PBMCs**

| | 2N18D10 +IMCR | 2N18D10h16+IMCR | 057G03 | IMC-C103C |
|---|---|---|---|---|
| EC₅₀ (n M) | 0.002358 | 0.002558 | 0.004529 | 0.01015 |

### 5.5.3 TCRm×CD3 bispecific antibody mediates the killing of HLA-A02⁺/MAGE-A4⁺ tumor cells via PBMCs

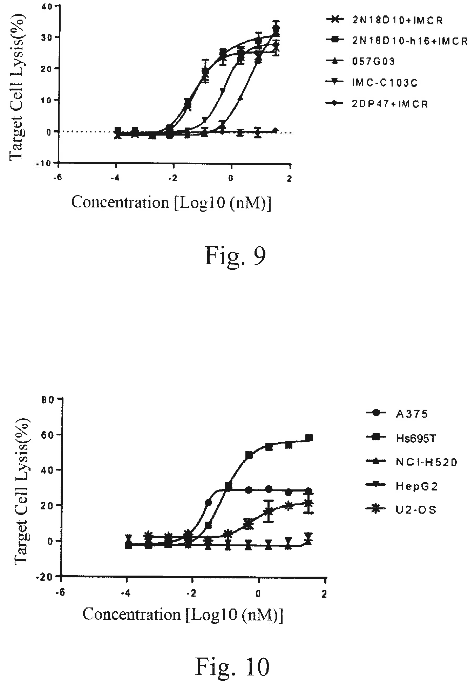

Antigen double-positive (HLA-A02⁺/MAGE-A4⁺) cells including A375 (human malignant melanoma cells), Hs695T (human malignant melanoma cells), and U2-OS (human osteosarcoma cells), as well as the negative control cells NIC-H520 (human lung squamous cell carcinoma cells, HLA-A02⁻/MAGE-A4⁺) and HepG2 (human liver cancer cells, HLA-A02⁺/MAGE-A4⁻) were collected. After digestion and centrifugation, the cells were resuspended in RPMI 1640 medium to a concentration of 4×10⁵ cells/mL. Then, 50 µL of the suspension was plated into each well of a plate. The bispecific antibody 2N18D10+IMCR or 2N18D10-h16+IMCR, at a starting concentration of 30 nM (with 4-fold dilution gradients, for a total of 10 concentrations), was added at 100 µL per well. The negative control sample 2DP47+IMCR and the positive control samples 057G03 and IMC-C103C were used at the same concentrations. PBMCs (effector cells) were resuspended to 4×10⁶ cells/mL. Then, 50 µL of the suspension was added into each well of the above plate, achieving a final E:T ratio of 10:1. A separate target cell control, a separate effector cell control (PBMCs), and a medium-only blank control were prepared. The volume of each well was adjusted to 200 µL with medium. After 24-48 hours of incubation, the supernatant was collected, and the TCRm×CD3 bispecific antibody-mediated killing of tumor cells via PBMCs was analyzed according to the CytoTox96^{®} Non-Radioactive Cytotoxicity Assay instructions (Promega, G1780). The results showed that 2N18D10-h16+IMCR could mediate the specific killing of tumor cells via PBMCs (FIG. 10), and 2N18D10+IMCR and 2N18D10h16+IMCR exhibited higher activity in mediating the killing of A375 cells than 057G03 and IMC-C103C (FIG. 9 and Table 9).

**Table 9. EC₅₀ values of TCRm×CD3 bispecific antibodies in mediating killing of A375 cells via PBMCs**

| | 2N18D10+IMCR | 2N18D10h16+IMCR | 057G03 | IMC-C103C |
|---|---|---|---|---|
| EC₅₀ (nM) | 0.04985 | 0.0761 | 5.52 | 0.5267 |

### Example 6: TCRm×CD3 bispecific antibody recognizes the key amino acid analysis of MAGE-A4₂₃₀₋₂₃₉ (GVYDGREHTV)

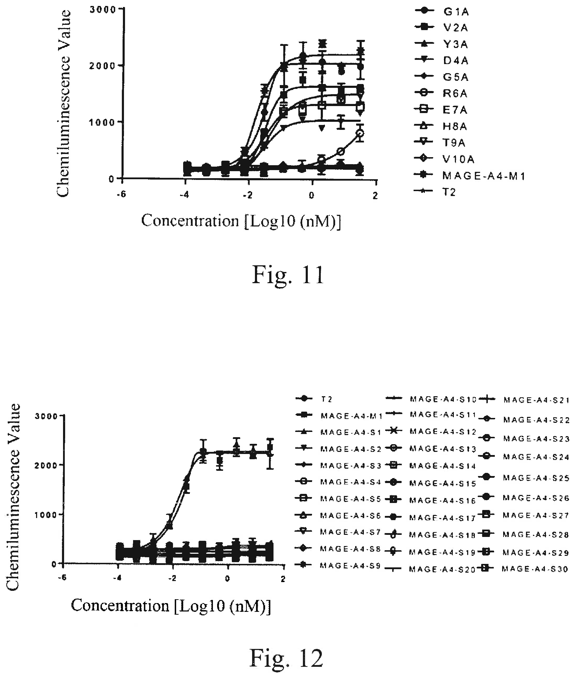

The alanine-scanning mutant peptide segments of MAGE-A4₂₃₀₋₂₃₉ (GVYDGREHTV) were chemically synthesized. T2 cells were harvested, centrifuged and resuspended in growth medium to a concentration of 1×10⁶ cells/mL. 1 mL of the suspension was plated into each well of a 24-well plate. The peptide segment MAGE-A4₂₃₀₋₂₃₉ (GVYDGREHTV) and its alanine-scanning mutant peptides were added to the 24-well plate at a final concentration of 50 µg/mL for overnight pulsing. After 16 hours, the cells were centrifuged and resuspended in RPMI 1640 medium to a concentration of 4×10⁵ cells/mL. 50 µL of the suspension was plated into each well of a 96-well plate. Jurkat-Dual cells were harvested, centrifuged and resuspended in RPMI1640 medium to a concentration of 4×10⁵ cells/mL. 50 µL of the suspension was added into each well of the plate to obtain a final E:T ratio of 1:1. Then, the bispecific antibody 2N18D10-h16+IMCR, at a starting final concentration of 30 nM (with 4-fold dilution gradients, for a total of 8 concentrations) was added at 100 µL per well. The negative control sample 2DP47+IMCR and the positive control samples 057G03 and IMC-C103C were used at the same concentrations. After 24 hours of incubation, the supernatant was collected, and the specific activation of Jurkat-Dual cells via different peptide-pulsed T2 cells were analysed according to the QUANTI-Luc^{™}instructions (QUANTI-Luc^{™}, Invivogen, rep-qlc2). The results showed that 2N18D10-h16+IMCR could not mediate the activation of Jurkat-Dual cells via alanine-scanning mutant peptides (at positions 3, 5, 6, 8, or 10)-pulsed T2 cells (FIG. 11), indicating that the amino acids at positions 3, 5, 6, 8, and 10 of MAGE-A4₂₃₀₋₂₃₉ (GVYDGREHTV) are the key amino acids for 2N18D10-h16+IMCR binding.

### Example 7: Interaction analysis of TCRm×CD3 bispecific antibody with similar peptides

The similar peptide segments of MAGE-A4₂₃₀₋₂₃₉ (GVYDGREHTV) in Table 10 were chemically synthesized.

**Table 10. Information on similar peptide segments of MAGE-A4₂₃₀₋₂₃₉ (GVYDGREHTV)**

| **SEQ ID NO:** | **Name of peptide segment** | **Sequence** | **Uniprot ID** | **Normal tissues presenting peptide segment** |
|---|---|---|---|---|
| 1 | MAGE-A4₂₃₀₋₂₃₉ | GVYDGREHTV | P43358 | |
| 2 | MAGE-A4-S1 | GLYDGREHSV | P43361 | |
| 3 | MAGE-A4-S2 | EVYDGREHSA | P43355 | |
| 4 | MAGE-A4-S3 | ISYSGVLHAV | 075165 | |
| 5 | MAGE-A4-S4 | GGYPGGIHEV | P35908 | |
| 6 | MAGE-A4-S5 | YLISQVEGHQV | Q9Y3C4 | bone marrow |
| 7 | MAGE-A4-S6 | DSYGGRHHRL | Q10570 | |
| 8 | MAGE-A4-S7 | VGYPGRLHLW | Q93063 | |
| 9 | MAGE-A4-S8 | FVYDEPGHAV | Q9NZM1 | adrenal gland, aorta, urinary bladder, cerebellum, lung, ovary, small intestine, thyroid gland, tongue, trachea, uterus |
| 10 | MAGE-A4-S9 | SQYSGQLHEV | Q9BXB5 | adrenal gland, kidney, lymph node, ovary, spleen, uterus |
| 11 | MAGE-A4-S10 | ALYGRLEVV | Q92625 | ovary |
| 12 | MAGE-A4-S11 | NTYEGRPIYV | P15085 | pancreas |
| 13 | MAGE-A4-S12 | VLYDQPRHV | Q93074 | ovary |
| 14 | MAGE-A4-S13 | KIYEGAYHV | Q99685 | ovary, spleen |
| 15 | MAGE-A4-S14 | RLYTGMHTV | Q8IWX5 | kidney, ovary |
| 16 | MAGE-A4-S15 | GVYAGRPLSV | Q9NWN3 | |
| 17 | MAGE-A4-S16 | GVYQGRVSAV | Q96F86 | |
| 18 | MAGE-A4-S 17 | AVLDGRELRV | Q01130 | |
| 19 | MAGE-A4-S18 | RLYDGLFKV | Q16531 | adrenal gland, cerebellum, kidney, lung, spleen, thymus, ovary |
| 20 | MAGE-A4-S19 | DVYDGRFLV | P22303 | cerebellum, thymus |
| 21 | MAGE-A4-S20 | SRYDGQIAV | P41226 | adrenal gland, lung, spleen, thymus |
| 22 | MAGE-A4-S21 | NRYDGIYKV | Q96PU4 | adrenal gland, aorta, urinary bladder, bone marrow, brain, cerebellum, colon, oesophagus, gallbladder, kidney, liver, lung, lymph node, ovary, pancreas, prostate, skin, small intestine, spleen, thyroid gland, tongue, trachea, uterus |
| 23 | MAGE-A4-S22 | NRYDGQVAV | P22314 | adrenal gland, aorta, bone marrow, brain, cerebellum, colon, oesophagus, kidney, liver, lung, lymph node, pancreas, small intestine, spleen, thyroid gland, tongue, trachea |
| 24 | MAGE-A4-S23 | LMYDGTKEV | 014896 | kidney |
| 25 | MAGE-A4-S24 | HVYDGKFLARV | P06276 | kidney |
| 26 | MAGE-A4-S25 | YIYDGELVSK | Q9Y2D2 | adrenal gland, aorta, bone marrow, colon, oesophagus, heart, kidney, liver, lung, ovary, small intestine, spleen, testis, thymus, thyroid gland, trachea, uterus |
| 27 | MAGE-A4-S26 | ATYDGQFMKL | Q13219 | bone marrow |
| 28 | MAGE-A4-S27 | FAYDGKDYL | P13747 | adrenal gland, aorta, urinary bladder, bone marrow, cerebrum, cerebellum, colon, oesophagus, heart, kidney, liver, lung, lymph node, muscle, bone marrow, ovary, pancreas, prostate, skin, small intestine, spleen, testis, thymus, thyroid gland, tongue, trachea, uterus |
| 29 | MAGE-A4-S28 | KLYDGFQYL | Q9UBF8 | adrenal gland, aorta, urinary bladder, bone marrow, cerebrum, cerebellum, colon, oesophagus, heart, kidney, liver, lung, lymph node, ovary, pancreas, small intestine, spleen, testis, thymus, thyroid gland, tongue, trachea, yterus |
| 30 | MAGE-A4-S29 | SVYDGKLLI | Q15165 | cerebellum, oesophagus, ovary, small intestine, spleen, thyroid |
| 31 | MAGE-A4-S30 | YAYDGKDYI | P01889 | |

T2 cells were harvested, centrifuged and resuspended in growth medium to a concentration of 1×10⁶ cells/mL. 1 mL of the suspension was plated into each well of a 24-well plate. The peptide segment MAGE-A4₂₃₀₋₂₃₉ (GVYDGREHTV) and similar peptide segments were added to the 24-well plate at a final concentration of 50 µg/mL for overnight pulsing. After 16 hours, the cells were centrifuged and resuspended in RPMI 1640 medium to a concentration of 4×10⁵ cells/mL. 50 µL of the suspension was plated into each well of a plate. Jurkat-Dual cells were harvested, centrifuged and resuspended in RPMI 1640 medium to a concentration of 4×10⁵ cells/mL. 50 µL of the suspension was added into each well of the plate to obtain a final E:T ratio of 1:1. Then, the bispecific antibody 2N18D10-h16+IMCR, at a starting final concentration of 30 nM (with 4-fold dilution gradients, for a total of 8 concentrations) was added at 100 µL per well. After 24 hours of incubation, the supernatant was collected, and the specific activation of Jurkat-Dual cells via similar peptide segments-pulsed T2 cells were analyzed according to the QUANTI-Luc^{™}instructions (QUANTI-Luc^{™}, Invivogen, rep-qlc2). The results, as shown in FIG. 12, demonstrated that in addition to specifically mediating the activation of Jurkat-Dual cells via MAGE-A4₂₃₀₋₂₃₉ (i.e., MAGE-A4-M1)-pulsed T2 cells, 2N18D10-h16+IMCR could also mediate the activation of Jurkat-Dual cells via cells pulsed with the same family sequence MAGE-A4-S1 (antigen peptide of MAGE-A8 presented by HLA-A02); but could not mediate the activation of Jurkat-Dual cells via T2 cells loaded with other similar peptide segments. MAGE-A8 also belongs to the MAGE family and has the same expression pattern as MAGE-A4, which is restrictively expressed in testicular and placental tissues in healthy individuals, but specifically expressed in some tumor tissues in cancer patients.

### Example 8: Interaction analysis of TCRm×CD3 bispecific antibody with different antigen-negative tumor cells

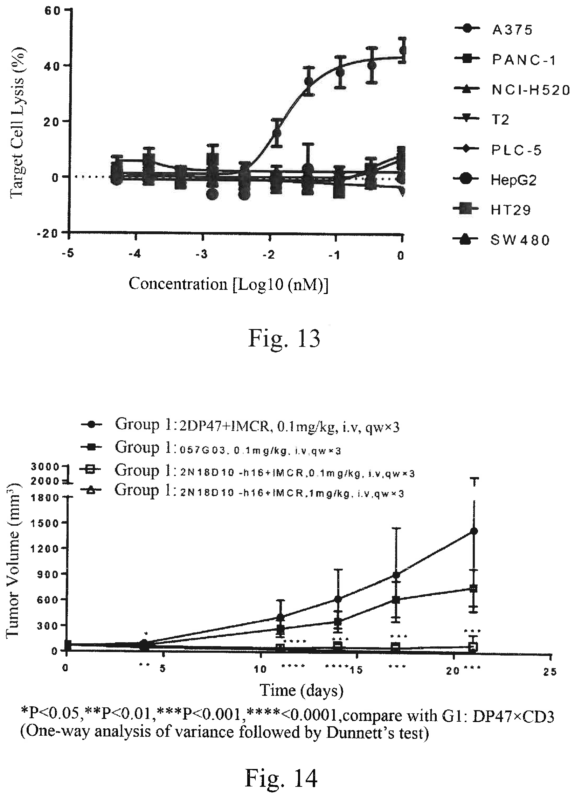

Human PBMCs were obtained by sorting following the method described in Section 5.5.1. The different tumor cells were collected, and the antigen information of the cells was shown in Table 11. Following the method described in Section 5.5.3, the killing assay of different antigen-negative tumor cells via PBMCs mediated by the bispecific antibody 2N18D10-h16+IMCR was performed. The results, as shown in FIG. 13, demonstrated that 2N18D10-h16+IMCR could only mediate the cytotoxicity of the HLA-A02⁺/MAGE-A4⁺ cells A375 via PBMCs, but could not mediate the killing of antigen-negative tumor cells via PBMCs.

**Table 11. Information on antigen expression in different tumor cells**

| Cells | HLA-A02 | MAGE-A4 | Cells | HLA-A02 | MAGE-A4 |
|---|---|---|---|---|---|
| A375 | + | + | SW480 | + | - |
| PANC-1 | + | - | HT29 | + | - |
| HepG2 | + | - | NCI-H520 | - | + |
| T2 | + | - | PLC/PRF/5 | - | - |

| | | | | | |
|---|---|---|---|---|---|
| Note: "+" means positive and "-" means negative. | | | | | |

### Example 9: Antitumor effect of TCRm×CD3 bispecific antibody in hPBMC immune reconstitution mice

Human PBMCs were obtained by sorting following the method described in Section 5.5.1.

Forty 5-6-week-old female NOG mice (Beijing Vitalstar Biotechnology Co., Ltd.) were selected. Each mouse was inoculated intraperitoneally with 8×10⁶ human PBMCs. On day 7 after human PBMC inoculation, NPG mice were inoculated subcutaneously on the right flank with 1×10⁷ A375 cells. When the average tumor volume reached 70 mm³, the mice were randomly assigned to groups based on tumor size, with the day of grouping defined as day 0, and administration was performed. The experiment was divided into four groups: the bispecific antibody 2N18D10-h16+IMCR groups, including 1 mg/kg and 0.1 mg/kg, 2DP47+IMCR negative control group, and 057G03 positive control group, with 6 mice in each group. The mice were administered by tail vein injection, once a week for three times, with continuous observation. Efficacy was evaluated according to the tumor growth inhibition (TGI)

The animals generally maintained a good mental state throughout the experiment. The tested bispecific antibody 2N18D10-h16+IMCR had a significant inhibitory effect on tumor growth at a dose of 0.1 mg/kg (Group 3), with a relative TGI (%) of 99.09%. Three animals had complete tumor regression, with very significant differences compared to the negative control group (Group 1) (p < 0.001). At a dose of 1 mg/kg (Group 4), 2N18D10-h16+IMCR had a more significant inhibitory effect on tumor growth, with a relative TGI (%) of 104.2%. Three animals had complete tumor regression, with very significant differences compared to the negative control group (Group 1) (p < 0.001). The bispecific antibody 2N18D10-h16+IMCR effectively inhibited tumor growth and showed superior efficacy compared to 057G03 (Group 2) (FIG. 14). No animals in the treated groups died, and no significant drug toxicity was observed.

All patents, patent application publications, and non-patent literature mentioned and/or listed in this application are incorporated herein by reference in their entirety. Exemplary embodiments of the inventions in the present application have been described hereinabove. However, those skilled in the art can modify or amend the exemplary embodiments described herein without departing from the spirit and scope of the present application, and variations or equivalents derived therefrom also fall within the scope of the present application.

### Sequence Listing

**SEQ ID NO: 1**
   GVYDGREHTV
**SEQ ID NO: 2**
   GLYDGREHSV
**SEQ ID NO: 3**
   EVYDGREHSA
**SEQ ID NO: 4**
   ISYSGVLHAV
**SEQ ID NO: 5**
   GGYPGGIHEV
**SEQ ID NO: 6**
   YLISQVEGHQV
**SEQ ID NO: 7**
   DSYGGRHHRL
**SEQ ID NO: 8**
   VGYPGRLHLW
**SEQ ID NO: 9**
   FVYDEPGHAV
**SEQ ID NO: 10**
   SQYSGQLHEV
**SEQ ID NO: 11**
   ALYGRLEVV
**SEQ ID NO: 12**
   NTYEGRPIYV
**SEQ ID NO: 13**
   NTYEGRPIYV
**SEQ ID NO: 14**
   KIYEGAYHV
**SEQ ID NO: 15**
   RLYTGMHTV
**SEQ ID NO: 16**
   GVYAGRPLSV
**SEQ ID NO: 17**
   GVYQGRVSAV
**SEQ ID NO: 18**
   AVLDGRELRV
**SEQ ID NO: 19**
   RLYDGLFKV
**SEQ ID NO: 20**
   DVYDGRFLV
**SEQ ID NO: 21**
   SRYDGQIAV
**SEQ ID NO: 22**
   NRYDGIYKV
**SEQ ID NO: 23**
   NRYDGQVAV
**SEQ ID NO: 24**
   LMYDGTKEV
**SEQ ID NO: 25**
   HVYDGKFLARV
**SEQ ID NO: 26**
   YIYDGELVSK
**SEQ ID NO: 27**
   ATYDGQFMKL
**SEQ ID NO: 28**
   FAYDGKDYL
**SEQ ID NO: 29**
   KLYDGFQYL
**SEQ ID NO: 30**
   SVYDGKLLI
**SEQ ID NO: 31**
   YAYDGKDYI
**SEQ ID NO: 32**
   DSDMG
**SEQ ID NO: 33**
   TITTDGSTYYADSVKG
**SEQ ID NO: 34**
   DLPILRPTPCGDSVLYNY
**SEQ ID NO: 35** 
EVQLVESGGGSVQTGETLRLSCTVSGFTFDDSDMGWYRQAPGNECELVSTITTDGSTYYADSVKGRFTIS
QDNAKTTAYLQMDSLKPEDTAVYYCAADLPILRPTPCGDSVLYNYWGQGTQVTVSS
**SEQ ID NO: 36** 
QVQLVESGGGLVKPGGSLRLSCAVSGFTFDDSDMGWYRQAPGNECELVSTITTDGSTYYADSVKGRFTIS
QDNAKTSAYLQMNSLRAEDTAVYYCAADLPILRPTPCGDSVLYNYWGQGTTVTVSS
**SEQ ID NO: 37**
   GYTMN
**SEQ ID NO: 38**
   LINPYKGVSTYNQKFKD
**SEQ ID NO: 39**
   SGYYGDSDWYFDV
**SEQ ID NO: 40**
   RASQDIRNYLN
**SEQ ID NO: 41**
   YTSRLES
**SEQ ID NO: 42**
   QQGNTLPWT
**SEQ ID NO: 43**
**SEQ ID NO: 44**
**SEQ ID NO: 45** 
QVQLVESGGGLVKPGGSLRLSCAVSGFTFDDSDMGWYRQAPGNECELVSTITTDGSTYYADSVKGRFTIS
QDNAKTSAYLQMNSLRAEDTAVYYCAADLPILRPTPCGDSVLYNYWGQGTTVTVSSGGGGSGGGGSGG
GGSQVQLVESGGGLVKPGGSLRLSCAVSGFTFDDSDMGWYRQAPGNECELVSTITTDGSTYYADSVKGR
FTISQDNAKTSAYLQMNSLRAEDTAVYYCAADLPILRPTPCGDSVLYNYWGQGTTVTVSSASERKSSVEC
PPCPAPEFEGGPSVFLFPPKPKDTLMISRTPEVTCVVVDVSQEDPEVQFNWYVDGVEVHNAKTKPREEQY
NSTYRVVSVLTVLHQDWLNGKEYKCKVSNKALPASIEKTISKAKGQPREPQVCTLPPSQEEMTKNQVSLS
CAVKGFYPSDIAVEWESNGQPENNYKTTPPVLDSDGSFFLVSKLTVDKSRWQEGNVFSCSVMHEALHNH
YTQKSLSLSPGK
**SEQ ID NO: 46** 
DIQMTQSPSSLSASVGDRVTITCRASQDIRNYLNWYQQKPGKAPKLLIYYTSRLESGVPSRFSGSGSGTDY
TLTISSLQPEDFATYYCQQGNTLPWTFGQGTKVEIKGGGGSGGGGSGGGGSGGGGSGGGSEVQLVESGG
GLVQPGGSLRLSCAASGYSFTGYTMNWVRQAPGKGLEWVALINPYKGVSTYNQKFKDRFTISVDKSKNT
AYLQMNSLRAEDTAVYYCARSGYYGDSDWYFDVWGQGTLVTVSSASERKSSVECPPCPAPEFEGGPSVF
LFPPKPKDTLMISRTPEVTCVVVDVSHEDPEVKFNWYVDGVEVHNAKTKPREEQYNSTYRVVSVLTVLH
QDWLNGKEYKCKVSNKALPASIEKTISKAKGQPREPQVYTLPPCREEMTKNQVSLWCLVKGFYPSDIAVE
WESNGQPENNYKTTPPVLDSDGSFFLYSKLTVDKSRWQQGNVFSCSVMHEALHNHYTQKSLSLSPGK
**SEQ ID NO: 47** 
GVYDGREHTVGCGGSGGGGSGGGGSIQRTPKIQVYSRHPAENGKSNFLNCYVSGFHPSDIEVDLLKNGE
RIEKVEHSDLSFSKDWSFYLLYYTEFTPTEKDEYACRVNHVTLSQPKIVKWDRDMGGGGSGGGGSGGGG
SGGGGSGSHSMRYFFTSVSRPGRGEPRFIAVGYVDDTQFVRFDSDAASQRMEPRAPWIEQEGPEYWDGE
TRKVKAHSQTHRVDLGTLRGCYNQSEAGSHTVQRMYGCDVGSDWRFLRGYHQYAYDGKDYIALKEDL
RSWTAADMAAQTTKHKWEAAHVAEQLRAYLEGTCVEWLRRYLENGKETLQRTDAPKTHMTHHAVSDH
EATLRCWALSFYPAEITLTWQRDGEDQTQDTELVETRPAGDGTFQKWAAVVVPSGQEQRYTCHVQHEGL
PKPLTLRWE
**SEQ ID NO: 48** 
MSSEQKSQHCKPEEGVEAQEEALGLVGAQAPTTEEQEAAVSSSSPLVPGTLEEVPAAESAGPPQSPQGASA
LPTTISFTCWRQPNEGSSSQEEEGPSTSPDAESLFREALSNKVDELAHFLLRKYRAKELVTKAEMLERVIK
NYKRCFPVIFGKASESLKMIFGIDVKEVDPASNTYTLVTCLGLSYDGLLGNNQIFPKTGLLIIVLGTIAMEG
DSASEEEIWEELGVMGVYDGREHTVYGEPRKLLTQDWVQENYLEYRQVPGSNPARYEFLWGPRALAET
SYVKVLEHVVRVNARVRIAYPSLREAALLEEEEGV
**SEQ ID NO: 49**
   GGGGSGGGGSGGGGS
**SEQ ID NO: 50**
   TCCTTCCCCGTCAGCCAGTCCT
**SEQ ID NO: 51** 
QDGNEEMGGITQTPYKVSISGTTVILTCPQYPGSEILWQHNDKNIGGDEDDKNIGSDEDHLSLKEFSELEQ
SGYYVCYPRGSKPEDANFYLYLRARVCENCMEMD
**SEQ ID NO: 52**
**SEQ ID NO: 53**
   HHHHHH
**SEQ ID NO: 54** 
ASEPKSSDKTHTCPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTVDKSRWQQGNV
FSCSVMHEALHNHYTQKSLSLSPGK
**SEQ ID NO: 55** 
ASPRGPTIKPCPPCKCPAPNLLGGPSVFIFPPKIKDVLMISLSPIVTCVVVDVSEDDPDVQISWFVNNVEVH
TAQTQTHREDYNSTLRVVSALPIQHQDWMSGKEFKCKVNNKDLPAPIERTISKPKGSVRAPQVYVLPPPE
EEMTKKQVTLTCMVTDFMPEDIYVEWTNNGKTELNYKNTEPVLDSDGSYFMYSKLRVEKKNWVERNS
YSCSVVHEGLHNHHTTKSFSRTPGK
**SEQ ID NO: 56** 
ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSREEMTKNQVSLTCLVKGFYPSDIAVEWESNGQPENNYKTTPPV
LDSDGSFFLYSKLTVDKSRWQQGNVFSCSVMHEALHNHYTQKSLSLSPGK
**SEQ ID NO: 57** 
ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNGKEYKCKVSNK
ALPAPIEKTISKAKGQPREPQVCTLPPSREEMTKNQVSLSCAVKGFYPSDIAVEWESNGQPENNYKTTPPV
LDSDGSFFLVSKLTVDKSRWQQGNVFSCSVMHEALHNHYTQKSLSLSPGK
**SEQ ID NO: 58** 
ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNGKEYKCKVSNK
ALPAPIEKTISKAKGQPREPQVYTLPPCREEMTKNQVSLWCLVKGFYPSDIAVEWESNGQPENNYKTTPPV
LDSDGSFFLYSKLTVDKSRWQQGNVFSCSVMHEALHNHYTQKSLSLSPGK
**SEQ ID NO: 59** 
ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPEFEGGPSVFLFPPKPKDTLMISRTPEVT
CVVVDVSHEDPEVKFNWYVDGVEVHNAKTKPREEQYNSTYRVVSVLTVLHQDWLNGKEYKCKVSNK
ALPASIEKTISKAKGQPREPQVCTLPPSREEMTKNQVSLSCAVKGFYPSDIAVEWESNGQPENNYKTTPPV
LDSDGSFFLVSKLTVDKSRWQQGNVFSCSVMHEALHNHYTQKSLSLSPGK
**SEQ ID NO: 60** ASTKGPSVFPLAPSSKSTSGGTAALGCLVKDYFPEPVTVSWNSGALTSGVHTFPAVLQSSGLYSLSSVVTV 
PSSSLGTQTYICNVNHKPSNTKVDKRVEPKSCDKTHTCPPCPAPEFEGGPSVFLFPPKPKDTLMISRTPEVT
CVVVDVSHEDPEVKFNWYVDGVEVHNAKTKPREEQYNSTYRVVSVLTVLHQDWLNGKEYKCKVSNK
ALPASIEKTISKAKGQPREPQVYTLPPCREEMTKNQVSLWCLVKGFYPSDIAVEWESNGQPENNYKTTPPV
LDSDGSFFLYSKLTVDKSRWQQGNVFSCSVMHEALHNHYTQKSLSLSPGK
**SEQ ID NO: 61** 
ASTKGPSVFPLAPSSKSTSEGTAALGCLVKDYFPEPVTVSWNSGALTSGVHTFPAVLQSSGLYSLSSVVTVP
SSSLGTQTYICNVDHKPSNTKVDKTVERKSCVECPPCPAPEFEGGPSVFLFPPKPKDTLMISRTPEVTCVVV
DVSQEDPEVQFNWYVDGVEVHNAKTKPREEQYNSTYRVVSVLTVLHQDWLNGKEYKCKVSNKALPASI
EKTISKAKGQPREPQVCTLPPSQEEMTKNQVSLSCAVKGFYPSDIAVEWESNGQPENNYKTTPPVLDSDG
SFFLVSKLTVDKSRWQEGNVFSCSVMHEALHNHYTQKSLSLSPGK
**SEQ ID NO: 62** 
ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKRVERKSCVECPPCPAPEFEGGPSVFLFPPKPKDTLMISRTPEVTCVV
VDVSHEDPEVKFNWYVDGVEVHNAKTKPREEQYNSTYRVVSVLTVLHQDWLNGKEYKCKVSNKALPA
SIEKTISKAKGQPREPQVYTLPPCREEMTKNQVSLWCLVKGFYPSDIAVEWESNGQPENNYKTTPPVLDS
DGSFFLYSKLTVDKSRWQQGNVFSCSVMHEALHNHYTQKSLSLSPGK
**SEQ ID NO: 63** 
VLGQPKAAPSVTLFPPSSEELQANKATLVCLISDFYPGAVTVAWKADSSPVKAGVETTTPSKQSNNKYAAS
SYLSLTPEQWKSHRSYSCQVTHEGSTVEKTVAPTECS
**SEQ ID NO: 64**
SEQ ID NO: 65 
EVQLLESGGGLVQPGGSLRLSCAASGFTFSSYAMSWVRQAPGKGLEWVSAISGSGGSTYYADSVKGRFTI
SRDNSKNTLYLQMNSLRAEDTAVYYCAKGSGFDYWGQGTLVTVSS
SEQ ID NO: 66 
EIVLTQSPGTLSLSPGERATLSCRASQSVSSSYLAWYQQKPGQAPRLLIYGASSRATGIPDRFSGSGSGTDFT
LTISRLEPEDFAVYYCQQYGSSPLTFGQGTKVEIK
SEQ ID NO: 67 
ANQVEQSPQSLIILEGKNVTLQCNYTVSPFSNLRWYKQDTGRGPVSLTILDYAINTKSNGRYTATLDADTK
QSSLHITASQLSDSASYICVVNRADGLYIPTFGRGTSLIVHP
SEQ ID NO: 68 
DVKVTQSSRYLVKRTGEKVFLECVQDAPLSKMFWYRQDPGLGLRLIYFSYDVKMKEKGDIPEGYSVSRE
KKERFSLILESASTNQTSMYLCASSSDQNSGDPYEQYFGPGTRLTVT
SEQ ID NO: 69 
ANQVEQSPQSLIILEGKNVTLQCNYTVSPFSNLRWYKQDTGRGPVSLTILDYAINTKSNGRYTATLDADTK
QSSLHITASQLSDSASYICVVNRADGLYIPTFGRGTSLIVHPYIQNPDPAVYQLRDSKSSDKFVCLFTDFDSQ
INVSQSKDSDVYITDKCVLDMRSMDFKSNSAVAWSNKSDFTCANAFNNSIIPEDTFFPSPESSCGGGGSAS
ERKSSVECPPCPAPEFEGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPASIEKTISKAKGQPREPQVYTLPPCREEM
TKNQVSLWCLVKGFYPSDIAVEWESNGQPENNYKTTPPVLDSDGSFFLYSKLTVDKSRWQQGNVFSCSV
MHEALHNHYTQKSLSLSPGK
**SEQ ID NO: 70** 
DVKVTQSSRYLVKRTGEKVFLECVQDAPLSKMFWYRQDPGLGLRLIYFSYDVKMKEKGDIPEGYSVSRE
KKERFSLILESASTNQTSMYLCASSSDQNSGDPYEQYFGPGTRLTVTEDLKNVFPPEVAVFEPSKAEISRTQ
KATLVCLATGFYPPHVELSWWVNGKEVHDGVCTDPQPLKEQPALNDSRYALSSRLRVSATFWQDPRNHF
 
RCQVQFYGLSENDEWTQDRAKPVTQIVSAEAWGRADCGGGGSASERKSCVECPPCPAPEFEGGPSVFLFP
PKPKDTLMISRTPEVTCVVVDVSQEDPEVQFNWYVDGVEVHNAKTKPREEQYNSTYRVVSVLTVLHQD
WLNGKEYKCKVSNKALPASIEKTISKAKGQPREPQVCTLPPSQEEMTKNQVSLSCAVKGFYPSDIAVEWE
SNGQPENNYKTTPPVLDSDGSFFLVSKLTVDKSRWQEGNVFSCSVMHEALHNHYTQKSLSLSPGK
**SEQ ID NO: 71** 
EVQLVESGGGSVQTGETLRLSCTVSGFTFDDSDMGWYRQAPGNECELVSTITTDGSTYYADSVKGRFTIS
QDNAKTTAYLQMDSLKPEDTAVYYCAADLPILRPTPCGDSVLYNYWGQGTQVTVSSGGGGSGGGGSGG
GGSEVQLVESGGGSVQTGETLRLSCTVSGFTFDDSDMGWYRQAPGNECELVSTITTDGSTYYADSVKGR
FTISQDNAKTTAYLQMDSLKPEDTAVYYCAADLPILRPTPCGDSVLYNYWGQGTQVTVSS
**SEQ ID NO: 72** 
QVQLVESGGGLVKPGGSLRLSCAVSGFTFDDSDMGWYRQAPGNECELVSTITTDGSTYYADSVKGRFTIS
QDNAKTSAYLQMNSLRAEDTAVYYCAADLPILRPTPCGDSVLYNYWGQGTTVTVSSGGGGSGGGGSGG
GGSQVQLVESGGGLVKPGGSLRLSCAVSGFTFDDSDMGWYRQAPGNECELVSTITTDGSTYYADSVKGR
FTISQDNAKTSAYLQMNSLRAEDTAVYYCAADLPILRPTPCGDSVLYNYWGQGTTVTVSS
**SEQ ID NO: 73** 
DIQMTQSPSSLSASVGDRVTITCRASQDIRNYLNWYQQKPGKAPKLLIYYTSRLESGVPSRFSGSGSGTDY
TLTISSLQPEDFATYYCQQGNTLPWTFGQGTKVEIKGGGGSGGGGSGGGGSGGGGSGGGSEVQLVESGG
GLVQPGGSLRLSCAASGYSFTGYTMNWVRQAPGKGLEWVALINPYKGVSTYNQKFKDRFTISVDKSKNT
AYLQMNSLRAEDTAVYYCARSGYYGDSDWYFDVWGQGTLVTVSS
**SEQ ID NO: 74** 
EVQLVESGGGSVQTGETLRLSCTVSGFTFDDSDMGWYRQAPGNECELVSTITTDGSTYYADSVKGRFTIS
QDNAKTTAYLQMDSLKPEDTAVYYCAADLPILRPTPCGDSVLYNYWGQGTQVTVSSGGGGSGGGGSGG
GGSEVQLVESGGGSVQTGETLRLSCTVSGFTFDDSDMGWYRQAPGNECELVSTITTDGSTYYADSVKGR
FTISQDNAKTTAYLQMDSLKPEDTAVYYCAADLPILRPTPCGDSVLYNYWGQGTQVTVSSASERKSSVEC
PPCPAPEFEGGPSVFLFPPKPKDTLMISRTPEVTCVVVDVSQEDPEVQFNWYVDGVEVHNAKTKPREEQY
NSTYRVVSVLTVLHQDWLNGKEYKCKVSNKALPASIEKTISKAKGQPREPQVCTLPPSQEEMTKNQVSLS
CAVKGFYPSDIAVEWESNGQPENNYKTTPPVLDSDGSFFLVSKLTVDKSRWQEGNVFSCSVMHEALHNH
YTQKSLSLSPGK
SEQ ID NO: 75 
DIQMTQSPSSLSASVGDRVTITCRASQDIRNYLNWYQQKPGKAPKLLIYYTSRLESGVPSRFSGSGSGTDY
TLTISSLQPEDFATYYCQQGNTLPWTFGQGTKVEIKGGGGSGGGGSGGGGSGGGGSGGGSEVQLVESGG
GLVQPGGSLRLSCAASGYSFTGYTMNWVRQAPGKGLEWVALINPYKGVSTYNQKFKDRFTISVDKSKNT
AYLQMNSLRAEDTAVYYCARSGYYGDSDWYFDVWGQGTLVTVSSGGGGSDVKVTQSSRYLVKRTGEK
VFLECVQDAPLSKMFWYRQDPGLGLRLIYFSYDVKMKEKGDIPEGYSVSREKKERFSLILESASTNQTSM
YLCASSSDQNSGDPYEQYFGPGTRLTVTEDLKNVFPPEVAVFEPSKAEISRTQKATLVCLATGFYPPHVELS
WWVNGKEVHDGVCTDPQPLKEQPALNDSRYALSSRLRVSATFWQDPRNHFRCQVQFYGLSENDEWTQD
RAKPVTQIVSAEAWGRADCGGGGSASERKSCVECPPCPAPEFEGGPSVFLFPPKPKDTLMISRTPEVTCVV
VDVSQEDPEVQFNWYVDGVEVHNAKTKPREEQYNSTYRVVSVLTVLHQDWLNGKEYKCKVSNKALPA
SIEKTISKAKGQPREPQVCTLPPSQEEMTKNQVSLSCAVKGFYPSDIAVEWESNGQPENNYKTTPPVLDSD
GSFFLVSKLTVDKSRWQEGNVFSCSVMHEALHNHYTQKSLSLSPGK
SEQ ID NO: 76 
AQLVESGGGLVQPGGSLRLSCAASAYFSFKAMSWVRQAPGKGLEWVGSISPSGGSTYYNDNVLGRFTIS
RDNSKNTLYLQMNSLRAEDTAVYYCAKDVGFFDEWGQGTLVTVSS
SEQ **ID** NO: 77 
DIQMTQSPSSLSASVGDRVTITCRASQSISSYLAWYQQKPGKAPKLLIYDASIRDIGVPSRFSGSGSGTDFT
LTISSLQPEDFATYYCQQYSSYPYTFGQGTKLEIK
**SEQ ID NO: 78** 
AQLVESGGGLVQPGGSLRLSCAASAYFSFKAMSWVRQAPGKGLEWVGSISPSGGSTYYNDNVLGRFTIS
RDNSKNTLYLQMNSLRAEDTAVYYCAKDVGFFDEWGQGTLVTVSSASTKGPSVFPLAPSSKSTSGGTAA
LGCLVEDYFPEPVTVSWNSGALTSGVHTFPAVLQSSGLYSLSSVVTVPSSSLGTQTYICNVNHKPSNTKVD
EKVEPKSCDGGGGSGGGGSDIQMTQSPSSLSASVGDRVTITCRASQDIRNYLNWYQQKPGKAPKLLIYYT
SRLESGVPSRFSGSGSGTDYTLTISSLQPEDFATYYCQQGNTLPWTFGQGTKVEIKSSASTKGPSVFPLAPS
SKSTSGGTAALGCLVKDYFPEPVTVSWNSGALTSGVHTFPAVLQSSGLYSLSSVVTVPSSSLGTQTYICNV
NHKPSNTKVDKRVERKSCVECPPCPAPEFEGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPASIEKTISKAKGQPR
EPQVYTLPPCREEMTKNQVSLWCLVKGFYPSDIAVEWESNGQPENNYKTTPPVLDSDGSFFLYSKLTVDK
SRWQQGNVFSCSVMHEALHNHYTQKSLSLSPGK
**SEQ ID NO: 79** 
AQLVESGGGLVQPGGSLRLSCAASAYFSFKAMSWVRQAPGKGLEWVGSISPSGGSTYYNDNVLGRFTIS
RDNSKNTLYLQMNSLRAEDTAVYYCAKDVGFFDEWGQGTLVTVSSASTKGPSVFPLAPSSKSTSGGTAA
LGCLVEDYFPEPVTVSWNSGALTSGVHTFPAVLQSSGLYSLSSVVTVPSSSLGTQTYICNVNHKPSNTKVD
EKVERKSCVECPPCPAPEFEGGPSVFLFPPKPKDTLMISRTPEVTCVVVDVSQEDPEVQFNWYVDGVEVH
NAKTKPREEQYNSTYRVVSVLTVLHQDWLNGKEYKCKVSNKALPASIEKTISKAKGQPREPQVCTLPPSQ
EEMTKNQVSLSCAVKGFYPSDIAVEWESNGQPENNYKTTPPVLDSDGSFFLVSKLTVDKSRWQEGNVFSC
SVMHEALHNHYTQKSLSLSPGK
**SEQ ID NO: 80** 
DIQMTQSPSSLSASVGDRVTITCRASQSISSYLAWYQQKPGKAPKLLIYDASIRDIGVPSRFSGSGSGTDFT
LTISSLQPEDFATYYCQQYSSYPYTFGQGTKLEIKRTVAAPSVFIFPPSDRKLKSGTASVVCLLNNFYPREA
KVQWKVDNALQSGNSQESVTEQDSKDSTYSLSSTLTLSKADYEKHKVYACEVTHQGLSSPVTKSFNRGE
C
**SEQ ID NO: 81** 
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSSASVAAPSVFIFPPSDE
QLKSGTASVVCLLNNFYPREAKVQWKVDNALQSGNSQESVTEQDSKDSTYSLSSTLTLSKADYEKHKVY
ACEVTHQGLSSPVTKSFNRGEC
**SEQ ID NO: 82**
   AVYDGREHTV
**SEQ ID NO: 83**
   GAYDGREHTV
**SEQ ID NO: 84**
   GVADGREHTV
**SEQ ID NO: 85**
   GVYAGREHTV
**SEQ ID NO: 86**
   GVYDAREHTV
**SEQ ID NO: 87**
   GVYDGAEHTV
**SEQ ID NO: 88**
   GVYDGRAHTV
**SEQ ID NO: 89**
   GVYDGREATV
**SEQ ID NO: 90**
   GVYDGREHAV
**SEQ ID NO: 91**
   GVYDGREHTA
**SEQ ID NO: 92** 
ASERKSSVECPPCPAPEFEGGPSVFLFPPKPKDTLMISRTPEVTCVVVDVSQEDPEVQFNWYVDGVEVHN
AKTKPREEQYNSTYRVVSVLTVLHQDWLNGKEYKCKVSNKALPASIEKTISKAKGQPREPQVCTLPPSQE
EMTKNQVSLSCAVKGFYPSDIAVEWESNGQPENNYKTTPPVLDSDGSFFLVSKLTVDKSRWQEGNVFSCS
VMHEALHNHYTQKSLSLSPGK
**SEQ ID NO: 93** 
ASERKSSVECPPCPAPEFEGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPASIEKTISKAKGQPREPQVYTLPPCRE
EMTKNQVSLWCLVKGFYPSDIAVEWESNGQPENNYKTTPPVLDSDGSFFLYSKLTVDKSRWQQGNVFSC
SVMHEALHNHYTQKSLSLSPGK
**SEQ ID NO: 94**

MLLGQKSQRYKAEEGLQAQGEAPGLMDVQIPTAEEQKAASSSSTLIMGTLEEVTDSGSPSPPQSPEGASSS
LTVTDSTLWSQSDEGSSSNEEEGPSTSPDPAHLESLFREALDEKVAELVRFLLRKYQIKEPVTKAEMLESVI
KNYKNHFPDIFSKASECMQVIFGIDVKEVDPAGHSYILVTCLGLSYDGLLGDDQSTPKTGLLIIVLGMILM
EGSRAPEEAIWEALSVMGLYDGREHSVYWKLRKLLTQEWVQENYLEYRQAPGSDPVRYEFLWGPRALA
ETSYVKVLEHVVRVNARVRISYPSLHEEALGEEKGV

### Reference

[1] Plaen, E.D., Arden, K., Traversarietal, C., et al. (1994) Structure, chromosomal localization, and expression of 12 genes of the MAGE family. Immunogenetics 40(5), 360-369.
[2] Rogner, U.C., Wilke, K., Steck, E., et al. (1994) The melanoma antigen gene (MAGE) family is clustered in the chromosomal band Xq28. Genomics 29(3), 725-731.
[3] Hao, Y.H., Doyle, J.M., Ramanathan, S., et al. (2013) Regulation of WASH-dependent actin polymerization and protein trafficking by ubiquitination. Cell 152, 1051-1064.
[4]. Chomez, P., De, B. O., Bertrand, M., et al. (2001).An overview of the mage gene family with the identification of all human members of the family. Cancer Research 61(14), 5544-5551.
[5] Simpson, A., Caballero, O.L., Jungbluth, A., et al. (2005) Cancer/testis antigens, gametogenesis and cancer. Nature Reviews Cancer 5(8), 615.
[6] Sharma, P., Shen, Y., Wen, S., et al. (2006) Cancer-testis antigens: expression and correlation with survival in human urothelial carcinoma. Clinical Cancer Research an Official Journal of the American Association for Cancer Research 12(18), 5442-5447.
[7] Bergeron, A., Valérie, P., Hélène, L., et al. (2010) High frequency of mage-a4 and mage-a9 expression in highrisk bladder cancer. International Journal of Cancer 125(6), 1365-1371.
[8] Peikert, T., Specks, U., Farver, C., et al., (2006) Melanoma antigen A4 is expressed in non-small cell lung cancers and promotes apoptosis. Cancer Research 66 (9), 4693-4700.
[9] Yakirevich, E., Sabo, E., Lavie, O., et al. (2003) Expression of the MAGE-A4 and NY-ESO-1 cancer-testis antigens in serous ovarian neoplasms. Clinical Cancer Research an Official Journal of the American Association for Cancer Research 9(17), 6453-6460.
[10] Quillien, V., Raoul, J. L., Heresbach, D., et al. (1997) Expression of MAGE genes in esophageal squamouscell carcinoma. Anticancer Research 17(1 a), 387-391.
[11] Ries, J., Schultze-Mosgau, S., Neukam,F., et al. (2005) Investigation of the expression of melanoma antigenencoding genes (MAGE-A1 to -A6) in oral squamous cell carcinomas to determine potential targets for gene-based cancer immunotherapy.International Journal of Oncology 26(3),817-824.
[12] M. T. Duffour, P. Chaux, C. Lurquin, G., et al. (1999) A MAGE-A4 peptide presented by HLA-A02 is recognized by cytolytic T lymphocytes. European Journal of Immunology 29(10),3329-3337.
[13]. Sanderson, J.P., Crowley, D.J., Wiedermann, G.E., et al. (2019) Preclinical evaluation of an affinity-enhanced MAGE-A4-specific T-cell receptor for adop-tive T-cell therapy. Oncoimmunology 9, 1682381.
[14] Ottaviani, S., Colau, D., Bruggen, P.V.D., et al. (2006) A new mage-4 antigen peptide recognized by cytolytict lymphocytes on HLA-A024 carcinoma cells. Cancer Immunology Immunotherapy 55(7), 867-872.
[15] Sung, H., Ferlay, J., Siegel, R.L., et al. (2021) Global Cancer Statistics 2020: GLOBOCAN Estimates of Incidence and Mortality Worldwide for 36 Cancers in 185 Countries. CA: a cancer journal for clinicians 71(3), 209-249.
[16] Kotsiou, E., Brzostek, J., Lenart, I., et al. (2010) Dimerization of soluble disulfide trap single-chain major histocompatibility complex class I molecules dependent on peptide binding affinity. Antioxid Redox Signal 15(3):635-644.
[17] Romao, E., Poignavent, V., Vincke, C., et al. (2018) Ritzenthaler C, Muyldermans S, Monsion B. Construction of High-Quality Camel Immune Antibody Libraries. Methods Mol Biol 1701:169-187.
[18] CN 201510097117.0
[19] Phage Display: A Practical Approach, Clackson, T. (USA) and Lowman, H. B. (USA) (ed.), translated by Lan Ma et al., Chemical Industry Press, May 2008.5.
[20] US 20160200833A1
[21] WO 2017175006A1
[22] Tan, P., Mitchell, D, A., Buss, T, N., et al. (2022) "Superhumanized" antibodies: reduction of immunogenic potential by complementarity-determining region grafting with human germline sequences: application to an anti-CD28. J Immunol 169(2):1119-1125.
[23] US 5821337A
[24] WO 2021122875A1

## Claims

1. A bispecific antibody comprising: a first antigen-binding fragment that binds to an HLA-A02/MAGE-A4 complex and/or an HLA-A02/MAGE-A8 complex, and a second antigen-binding fragment that binds to an activated T-cell antigen;
preferably, the first antigen-binding fragment binds to the HLA-A02/MAGE-A4 complex at an epitope comprising amino acids at positions 230-239 of MAGE-A4 as set forth in SEQ ID NO: 48;
more preferably, the first antigen-binding fragment binds to the HLA-A02/MAGE-A4 complex at an epitope comprising at least one of amino acids at positions 232, 234, 235, 237, and 239 of MAGE-A4 as set forth in SEQ ID NO: 48; and/or
the first antigen-binding fragment binds to the HLA-A02/MAGE-A8 complex at an epitope comprising amino acids at positions 232-241 of MAGE-A8 as set forth in SEQ ID NO: 94; and/or
preferably, the activated T-cell antigen is a CD3 molecule.

2. The bispecific antibody of claim 1, wherein
the first antigen-binding fragment comprises HCDR1 as set forth in SEQ ID NO: 32, HCDR2 as set forth in SEQ ID NO: 33, and HCDR3 as set forth in SEQ ID NO: 34;
preferably, the first antigen-binding fragment is in the form of a single-domain antibody; more preferably, the first antigen-binding fragment comprises a monovalent or multivalent single-domain antibody that binds to the HLA-A02/MAGE-A4 complex and/or HLA-A02/MAGE-A8 complex; and/or
the second antigen-binding fragment comprises HCDR1 as set forth in SEQ ID NO: 37, HCDR2 as set forth in SEQ ID NO: 38, HCDR3 as set forth in SEQ ID NO: 39, LCDR1 as set forth in SEQ ID NO: 40, LCDR2 as set forth in SEQ ID NO: 41, and LCDR3 as set forth in SEQ ID NO: 42;
preferably, the second antigen-binding fragment is a single-chain variable fragment (scFv) or a Fab fragment; more preferably, the second antigen-binding fragment is an scFv;
wherein the amino acid sequences of the HCDRs are defined according to Kabat numbering scheme.

3. The bispecific antibody of claim 1 or 2, wherein the first antigen-binding fragment comprises a divalent single-domain antibody that binds to the HLA-A02/MAGE-A4 complex and/or HLA-A02/MAGE-A8 complex;
optionally, the first antigen-binding fragment comprises two heavy chain variable regions of monovalent single-domain antibodies that bind to the HLA-A02/MAGE-A4 complex and/or HLA-A02/MAGE-A8 complex;
preferably, the first antigen-binding fragment comprises two heavy chain variable regions of monovalent single-domain antibodies that bind to the HLA-A02/MAGE-A4 complex and/or HLA-A02/MAGE-A8 complex, linked via a linker; more preferably, the linker is (GGGGS)n, where n is an integer ≥ 1, such as GGGGSGGGGSGGGGS (SEQ ID NO:49).

4. The bispecific antibody of any one of claims 1-3, wherein
the first antigen-binding fragment comprises the amino acid sequence as set forth in SEQ ID NOs: 35, 36, 71, or 72; and/or
the second antigen-binding fragment comprises a heavy chain variable region having the amino acid sequence as set forth in SEQ ID NO: 43 and a light chain variable region having the amino acid sequence as set forth in SEQ ID NO: 44;
preferably, the first antigen-binding fragment comprises the amino acid sequence as set forth in SEQ ID NO: 35; and the second antigen-binding fragment comprises a heavy chain variable region having the amino acid sequence as set forth in SEQ ID NO: 43 and a light chain variable region having the amino acid sequence as set forth in SEQ ID NO: 44; or
the first antigen-binding fragment comprises the amino acid sequence as set forth in SEQ ID NO: 36; and the second antigen-binding fragment comprises a heavy chain variable region having the amino acid sequence as set forth in SEQ ID NO: 43 and a light chain variable region having the amino acid sequence as set forth in SEQ ID NO: 44; or
the first antigen-binding fragment comprises the amino acid sequence as set forth in SEQ ID NO: 71; and the second antigen-binding fragment comprises a heavy chain variable region having the amino acid sequence as set forth in SEQ ID NO: 43 and a light chain variable region having the amino acid sequence as set forth in SEQ ID NO: 44; or
the first antigen-binding fragment comprises the amino acid sequence as set forth in SEQ ID NO: 72; and the second antigen-binding fragment comprises a heavy chain variable region having the amino acid sequence as set forth in SEQ ID NO: 43 and a light chain variable region having the amino acid sequence as set forth in SEQ ID NO: 44.

5. The bispecific antibody of any one of claims 1-4, wherein
the first antigen-binding fragment and the second antigen-binding fragment are linked via an Fc fragment of a heavy chain constant region comprising a first Fc fragment and a second Fc fragment;
preferably, the Fc fragment of the heavy chain constant region is an Fc fragment of IgG1 subtype;
more preferably, the Fc fragment of the heavy chain constant region is an Fc fragment of IgG1m3 allotype;
wherein the first Fc fragment comprises the amino acid C at position 354 and the amino acid W at position 366; or the amino acid C at position 349, the amino acid S at position 366, the amino acid A at position 368, and the amino acid V at position 407; and the second Fc fragment comprises the amino acid C at position 354 and the amino acid W at position 366; or the amino acid C at position 349, the amino acid S at position 366, the amino acid A at position 368, and the amino acid V at position 407;
preferably, the first Fc fragment comprises the amino acid C at position 354 and the amino acid W at position 366, and the second Fc fragment comprises the amino acid C at position 349, the amino acid S at position 366, the amino acid A at position 368, and the amino acid V at position 407; and/or
the first Fc fragment and the second Fc fragment comprise the amino acid F at position 234, the amino acid E at position 235 and the amino acid S at position 331, respectively; and/or
one of the first Fc fragment and the second Fc fragment is linked to the first antigen-binding fragment, and the other of the first Fc fragment and the second Fc fragment is linked to the second antigen-binding fragment;
wherein the amino acid positions of the antibody constant regions are determined according to EU numbering scheme.

6. The bispecific antibody of any one of claims 1-5, comprising a first arm that binds to the HLA-A02/MAGE-A4 complex and/or HLA-A02/MAGE-A8 complex and a second arm that binds to the activated T-cell antigen, wherein the first arm comprises the amino acid sequence as set forth in SEQ ID NOs: 45 or 74; and the second arm comprises the amino acid sequence as set forth in SEQ ID NO: 46.

7. A single-domain antibody that binds to an HLA-A02/MAGE-A4 complex and/or an HLA-A02/MAGE-A8 complex, comprising
HCDR1 as set forth in SEQ ID NO: 32,
HCDR2 as set forth in SEQ ID NO: 33, and
HCDR3 as set forth in SEQ ID NO: 34;
preferably, the single-domain antibody comprises the amino acid sequence as set forth in SEQ ID NOs: 35, 36, 71, or 72;
wherein the amino acid sequences of the HCDRs are defined according to Kabat numbering scheme.

8. A nucleic acid molecule encoding the bispecific antibody of any one of claims 1-6 or the single-domain antibody of claim 7.

9. A pharmaceutical composition comprising the bispecific antibody of any one of claims 1-6 or the single-domain antibody of claim 7, and a pharmaceutically acceptable excipient, diluent, or carrier.

10. The pharmaceutical composition of claim 9, for use in the prevention or treatment of an HLA-A02/MAGE-A4-positive tumor and/or an HLA-A02/MAGE-A8-positive tumor;
preferably, the HLA-A02/MAGE-A4-positive tumor and/or the HLA-A02/MAGE-A8-positive tumor are selected from the group consisting of esophageal cancer, urothelial carcinoma, oral carcinoma, sarcoma, head and neck cancer, lung cancer, liver cancer, bladder carcer, melanoma, ovarian cancer, colorectal carcinoma, and breast cancer.

11. Use of the bispecific antibody of any one of claims 1-6, the single-domain antibody of claim 7, or the pharmaceutical composition of claim 9 or 10 in the manufacture of a medicament for the prevention or treatment of an HLA-A02/MAGE-A4 positive tumor and/or an HLA-A02/MAGE-A8 positive tumor;
preferably, the HLA-A02/MAGE-A4 positive tumor and/or the HLA-A02/MAGE-A8 positive tumor are selected from the group consisting of esophageal cancer, urothelial carcinoma, oral carcinoma, sarcoma, head and neck cancer, lung cancer, liver cancer, bladder cancer, melanoma, ovarian cancer, colorectal cancer, and breast cancer.

12. A method for preventing or treating an HLA-A02/MAGE-A4 positive tumor and/or an HLA-A02/MAGE-A8 positive tumor, comprising administering to a subject in need thereof the bispecific antibody of any one of claims 1 to 6, the single-domain antibody of claim 7, or the pharmaceutical composition of claim 9 or 10;
preferably, the HLA-A02/MAGE-A4 positive tumor and/or the HLA-A02/MAGE-A8 positive tumor are selected from the group consisting of esophageal cancer, urothelial carcinoma, oral carcinoma, sarcoma, head and neck cancer, lung cancer, liver cancer, bladder cancer, melanoma, ovarian cancer, colorectal cancer, and breast cancer.
